# EUROPEAN PATENT SPECIFICATION

(11) **EP 1 244 668 B1**
(45) Date of publication and mention of the grant of the patent: **05.04.2006**
(21) Application number: 01907418.6
(22) Date of filing: 08.01.2001
(51) Int. Cl.: C07D 473/34, C07D 473/40, A61K 31/52, A61P 35/00

(54) **PURINE DERIVATIVES, PROCESS FOR THEIR PREPARATION AND USE THEREOF**
PURIN DERIVATE, IHRE HERSTELLUNG UND VERWENDUNG
DERIVES DE PURINE, PROCEDE DE PREPARATION ET UTILISATION DE CES DERNIERS

(30) Priority: 07.01.2000 EP 00200070
(43) Date of publication of application: 02.10.2002
(73) Proprietor: UNIVERSITAIRE INSTELLING ANTWERPEN, B-2610 Antwerp (BE); Ustav Experimentalni Botaniky Akademie ved Ceské Republiky, 160 00 Praha 6 (CZ)
(72) Inventor: HAVLICEK,Libor, 142 20 Praha (CZ); KRYSTOF, Vladimir;, 702 00 Ostrava 1 (CZ); SIGLEROV , Vera;, 120 00 Praha (CZ); LENOBEL, René, 785 01 Sternberk (CZ); VAN ONCKELEN, Henri, B-2100 Antwerp (BE); BERNEMAN, Zwi, Nisan, B-2018 Antwerp (BE); SLEGERS, Herman, B-2243 Pulle-Zandhoven (BE); ESMANS, Edgard, B-2000 Antwerp (BE); STRNAD, Miroslav, 779 00 Olomouc (CZ); VERMEULEN, Katrien, B-9150 Kruibeke (BE)
(74) Representative: 't Jong, Bastiaan Jacobus
(86) International application number: PCT/EP2001/000150
(87) International publication number: WO 2001/049688

(56) References cited:
- WO-A-97/16452
- WO-A-97/20842
- WO-A-99/07705
- US-A- 5 866 702
- VORBRUEGGEN, HELMUT ET AL: "N6-[2-(3,4-dihydroxyphenyl)ethyl]adenosin e. Amination of inosine" NUCLEIC ACID CHEM. (1978), VOLUME 2, 533-5. EDITOR(S): TOWNSEND, LEROY B.;TIPSON, R. STUART. PUBLISHER: WILEY, NEW YORK, N. Y. , XP001000046
- HAMILTON, H. W. ET AL: "Correlation of adenosine receptor affinities and cardiovascular activity" LIFE SCI. (1987), 41(20), 2295-302 , XP001000032
- NUGIEL, DAVID A. ET AL: "Facile Preparation of 2,6-Disubstituted Purines Using Solid-Phase Chemistry" J. ORG. CHEM. (1997), 62(1), 201-203 , XP001000071
- PATERSON, ALAN R. P. ET AL: "Inhibition by nitrobenzylthioinosine of adenosine uptake by asynchronous HeLa cells" MOL. PHARMACOL. (1977), 13(6), 1147-58 , XP001000020
- BRESSI, JEROME C. ET AL: "Adenosine Analogues as Inhibitors of Trypanosoma brucei Phosphoglycerate Kinase: Elucidation of a Novel Binding Mode for a 2-Amino-N6-Substituted Adenosine" J. MED. CHEM. (2000), 43(22), 4135-4150 , XP000999137
- SCHILLINGER, EKKEHARD ET AL: "Metabolic effects of N6-substituted adenosines in rats" BIOCHEM. PHARMACOL. (1974), 23(16), 2283-9 , XP001000021

## Description

The present invention relates to new purine derivatives and to their use in suitable utilities, in particular, diagnostic- and therapeutic methods.

The invention relates in particular to purine derivatives with an inhibitor effect with respect to cyclin-dependent kinase proteins (cdks) and also with an inhibitory effect with respect to viruses and immunostimulation.

The use of 2,6,9-trisubstituted purine derivatives as cdk-inhibitor is for example disclosed in WO 97/16452, WO 98/05335, WO/9720842, WO 97/16542, WO98/05335, WO 98/39007, WO 98/49146, WO 99/07705 and US 5,866,702.

Nucleotide analogues containing phosphonate groups are for example disclosed in U.S. patents 4,659,825; 4,724,233; 5,124,051; 5,302,585; 5,208,221; 5,352,786; 5,356,886; 5,142,051; in EP publication numbers 269,947; 481,214; 630,381; 369,409; 454,427; 618,214; 398,231; 454,427; 468,119; 481,119; 481,214; 434,450 and in WO 95/07920; WO 094/03467, WO96/33200 and WO94/03467. The typical purine base is adenine, 2,6-diaminopurine and guanine. The purine bases may include the aza- and deaza -analogues thereof. 6,9-Substituted and 2,6,9-trisubstituted purines and related analogues are disclosed in WO 96/33200. However, the selectivity and efficiency of these compounds when used for example as anticancer or anti-inflammatory agents has not been satisfactory.

It is the object of this invention to provide anticancer, anti-inflammatory, antiviral, antineuro-degenerative, neurodepressive and immunosuppressive compounds having improved selectivity and efficiency index, i.e. compounds that are less toxic, yet more efficacious than derivatives known heretofore. This object is achieved by the present invention by providing 2,6,9-trisubstituted purine derivatives according to claim 1 and the pharmaceutically acceptable salts thereof. The purine derivatives according to the present invention are useful, for example, for inhibiting cdk-activity and also for inhibiting cell proliferation and/or inducing apoptosis.

The present invention also provides methods for preparing said purine derivatives.

The inventions further relates to the use of the purine derivatives in methods for treatment of the human and animal body.

In addition, the invention provides pharmaceutical compositions comprising as the active ingredient one or more of the purine derivatives, together with at least a pharmaceutically acceptable carrier or diluent.

### SUMMARY OF THE INVENTION

The present invention provides 2,6,9-trisubstituted purine derivatives represented by general formula I: 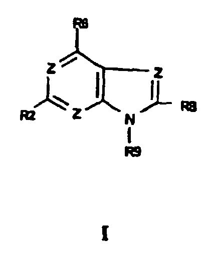 and pharmaceutically acceptable salts thereof, wherein:
- **Z**: is N;
- **R6**: is **R6'-X** wherein **X** is -NH-; and R6' is substituted arylalkyl,
- **R8**: is H,
- **R2**: is **R2**⁻- X wherein
**X** is -NH-,
**R2'** is (substituted) alkyl, and
- **R9**: is (substituted) alkyl

The purine derivatives of the invention preferably are used as an inhibitor of cyclin-dependent kinase proteins (cdks), as an antiviral, antimitotic, antiproliferative, immunomodulating, immune-suppressive, anti-inflammatory and/or antitumor agent. In addition, the purine derivatives of the invention can be used as modulator of β-adrenergic and/or purinergic receptors, as an inhibitor of proliferation of hematopoietic cells and cancer cells, and/or an inducer of apoptosis in cancer cells.

In a preferred embodiment the invention provides purine derivatives for use in the treatment of the human or animal body.

In another preferred embodiment the invention provides a pharmaceutical composition comprising one or more purine derivatives of the invention and a pharmaceutically acceptable carrier or diluent.

The purine derivatives according to the present invention are furthermore preferably used for several other applications, such as for the preparation of affinity absorption matrices.

### DETAILED DESCRIPTION OF THE INVENTION

As used herein, unless modified by the immediate context:
"Halogen" refers to fluorine, bromine, chlorine and iodine atoms.
"Hydroxyl" refers to -OH.
"Mercapto" refers to -SH.
"Alkyl" refers to a branched or unbranched C₁-C₆ chain which may be saturated or unsaturated, such as for example methyl, propyl, isopropyl, tert-butyl, allyl, vinyl, ethinyl, propargyl, or hexen-2-yl.
"Substituted alkyl" refers to an alkyl as defined above, including one or more substituents such as hydroxyl, mercapto, alkylmercapto, halogen, alkoxy, amino, amido, carboxyl, sulfo, acyl and the like. These groups may be attached to any carbon atom of the alkyl moiety.
"Alkoxy" refers to -OR, wherein R is (substituted) alkyl, (substituted) aryl, (substituted) arylalkyl, (substituted) cycloalkyl, (substituted) cycloheteroalkyl as defined.
"Alkylmercapto" relates to -SR, wherein R is as defined for "alkoxy".
"Sulfo" refers to -SO₃R, wherein R is H, alkyl or substituted alkyl.
"Sulfamido" refers to the group -NHSO₃R, wherein R is H, alkyl or substituted alkyl.
"Acyl" refers to -C(O)R, wherein R is hydrogen, (substituted) alkyl, (substituted) aryl, (substituted) arylalkyl, (substituted) cycloalkyl as defined herein. "Aryloxy" refers to groups -OAr, wherein Ar is (substituted aryl), or (substituted) heteroaryl group as defined herein.
"Alkylamino" refers to the group -NRR', wherein R and R' may independently be hydrogen, (substituted) alkyl, (substituted) aryl, or (substituted) heteroaryl as defined herein.
"Amido" refers to the group -C(O)NRR', wherein R and R' may independently be hydrogen, (substituted) alkyl, (substituted) aryl, or (substituted) heteroaryl as defined herein.
"Carboxyl" refers to the group -C(O)OR, wherein R is hydrogen, (substituted) alkyl, (substituted) aryl, or (substituted) heteroaryl as defined herein.
"Carbamino" refers to the group -NHCOR, wherein R is hydrogen, (substituted) alkyl, heterocycle, (substituted) aryl, or (substituted) heteroaryl as defined herein. "Aryl" or "Ar" refers to an aromatic carbocyclic group having at least one aromatic ring (e.g., phenyl or biphenyl) or multiple condensed rings in which at least one ring is aromatic (e.g., 1,2,3,4-tetrahydronaphthyl, naphthyl, anthryl, or phenanthryl).
"Substituted aryl" refers to aryl as defined above, optionally substituted with one or more functional groups such as halogen, alkyl, hydroxyl, amino, mercapto, alkoxy, alkylmercapto, alkylamino, amido, carboxyl, nitro, sulfo and the like.
"Heterocycle" refers to an unsaturated or aromatic carbocyclic group having at least one heteroatom, such as N, O or S, within the ring; the ring can be single condensed (e.g. pyranyl, pyridyl or furyl) or multiple condensed (e.g., quinazolinyl, purinyl, quinolinyl or benzofuranyl), which can optionally be substituted with, e.g., halogen, alkyl, alkoxy, alkylmercapto, alkylamino, amido, carboxyl, hydroxyl, nitro, mercapto, sulfo and the like.
"Heteroaryl" refers to a heterocycle in which at least one heterocyclic ring is aromatic.
"Substituted heteroaryl" refers to a heterocycle which optionally is monosubstituted or polysubstituted with one or more functional groups, e.g., halogen, alkyl, alkoxy, alkylthio, alkylamino, amido, carboxyl, hydroxyl, nitro, mercapto, sulfo and the like.
"(Substituted) arylalkyl" refers to the group -R-Ar wherein Ar is an aryl group and R is alkyl or substituted alkyl group. The aryl groups are optionally substituted with, e.g., halogen, alkyl, hydroxyl, alkoxy, alkylmercapto, alkylamino, amido, carboxyl, hydroxy, aryl, nitro, mercapto, sulfo and the like.
"(Substituted) heteroalkyl" refers to the group -R-Het, wherein Het is a heterocycle group and R is an alkyl group. The heteroalkyl groups are optionally substituted with e.g., halogen, alkyl, alkoxy, alkylthio, alkylamino, amido, carboxy, alkoxycarbonyl, aryl, aryloxy, nitro, thiol, sulfonyl and the like.
"(Substituted) heteroarylalkyl" refers to the group -R-HetAr wherein HetAr is a heteroaryl group and R is alkyl or substituted alkyl. Heteroarylalkyl groups are optionally substituted with, e.g., halogen, alkyl, substituted alkyl, alkoxy, alkylmercapto, nitro, thiol, sulfo and the like.
"Cycloalkyl" refers to a divalent cyclic or polycyclic alkyl group containing 3 to 15 carbon atoms.
"Substituted cycloalkyl" refers to a cycloalkyl group comprising one or more substituents such as, e.g., halogen, alkyl, substituted alkyl, alkoxy, alkylmercapto, aryl, nitro, mercapto, sulfo and the like. "Cycloheteroalkyl" refers to a cycloalkyl group wherein one or more of the ring carbon atoms is replaced with a heteroatom (e.g., N, O, S or P).
"Substituted cycloheteroalkyl" refers to a cycloheteroalkyl group as defined above, which contains one or more substituents, such as halogen, alkyl, alkoxy, alkylmercapto, alkylamino, amido, carboxyl, hydroxy, nitro, mercapto, sulfo and the like.
"(Substituted) cycloalkyl alkyl" refers to the group -R-cycloalkyl wherein cycloalkyl is a cycloalkyl group and R is an alkyl or substituted alkyl. The cycloalkyl group can optionally be substituted with e.g., halogen, alkyl, alkoxy, alkylmercapto, alkylamino, amido, carboxyl, hydroxy, nitro, mercapto, sulfo and the like. "(Substituted) cycloheteroalkyl alkyl" refers to -R-cycloheteroalkyl wherein R is alkyl or substituted alkyl. The cycloheteroalkyl group can optionally be substituted with e.g. halogen, alkyl, alkoxy, alkylmercapto, alkylamino, amido, carboxyl, hydroxy, nitro, mercapto, sulfo and the like.
"An amine substituted with a catechol group or related group" refers to secondary and tertiary amines containing at least one dihydroxyaryl or dihydroxyarylalkyl group".

The present invention provides purine derivatives represented by general formula I: 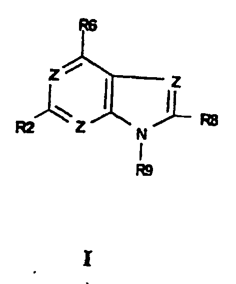 and pharmaceutically acceptable salts thereof, wherein:
- **Z**: is N R6 is a substituted -NH-phenylalkyl, wherein alkyl is a branched or linear, saturated or unsaturated C₁-C₆ lower alkyl such as methyl, ethyl, propyl, isopropyl, vinyl, propinyl, or propenyl, wherein the phenyl ring or the alkyl is substituted by 1 to 4 substituents, said substituents for the phenyl ring being selected from hydroxyl substituents and for the alkyl independently selected from halogen, such as chloro or fluoro, hydroxyl, amino, carboxyl or amido.
- **R2**: is **R2'-X**, wherein **X** is -NH-, ; and **R2'** is -C₃-C₆ branched or linear, saturated or unsaturated alkyl, such as, isopropyl, butyl, isobutyl, vinyl, allyl, propenyl, propargyl, propinyl, isopentenyl, or isobutenyl, optionally substituted by 1 to 3 substituents, selected from amino, or hydroxyl,
- **R8**: is H; and R9 is a branched or unbranched C₁-C₆ chain which may be saturated or unsaturated, such as for example methyl, propyl, isopropyl, tert-butyl, allyl, vinyl, ethinyl, propargyl, or hexen-2-yl.

The following purine derivatives are particularly preferred: 2-(1-hydroxymethylpropylamino)-6-(3,4-dihydroxybenzyl)amino-9-isopropylpurine, 2-(2-aminopropylamino)-6-(3,4-dihydroxybenzyl)amino-9-isopropylpurine, 2-(2-hydroxypropylamino)-6-(3,4-dihydroxybenzyl)-amino-9-isopropylpurine, 2-(R)-(1-isopropyl-2-hydroxyethylamino)-6-(3,4-dihydroxybenzyl)amino-9-isopropylpurine, 2-(2-hydroxypropylamino)-6-[1-(3,4-dihydroxyphenyl)ethyl]amino-9-isopropylpurine, 2-(R)-(1-isopropyl-2-hydroxyethylamino-6-[1-(3,4-dihydroxyphenyl) ethyl] amino-9-isopropylpurine, 2-(1R-isopropyl-2-hydroxyethylamino)-6-[(R)-(1-phenyl-2-hydroxyethyl)amino]-9-isopropylpurine ]purine, 2-(R)-(1-isopropyl-2-hydroxyethylamino-6-[(R,S)-(1-phenyl-2-hydroxyethyl)amino]-isopropylpurine,

The invention also relates to optical isomers and racemic mixtures, and, as the case may be, geometric isomers of the above-defined derivatives, in particular the (R) or (S) isomers of 2-(R)-(1-isopropyl-2-hydroxyethylamino)-6-[1-(3,4-dihydroxyphenyl)ethyl]amino-9-isopropylpurine, 2-(R)-(1-isopropyl-2-hydroxyethylamino)-6-(3,4-dihydroxybenzyl)amino-9-isopropylpurine, 2-(1R-isopropyl-2-hydroxyethylamino)-6-[(S)-(1-phenyl-2-hydroxyethyl)amino]-9-isopropylpurine, 2-(1S-isopropyl-2-hydroxyethylamino)-6-[(S)-(1-phenyl-2-hydroxyethyl)amino]-9-isopropylpurine.

The purine derivatives of the invention per se, or as intermediates in the preparation of novel compounds, have a wide variety of diagnostic, therapeutic and industrial utilities.

The compounds of this invention are for example suitable as intermediates for use in the preparation of affinity absorption matrices that harness the chemical properties of the compound's substituent groups. For example, the phosphonate groups in matrix-bound form are useful in the chromatographic separation of positively charged molecules. Other immobilised forms of the purine derivatives of the invention are for example useful in purifying proteins, e.g., cell cycle enzymes (such as cdks), or enzymes involved in the recognition of the compounds of this invention, e.g. transport proteins. Suitable methods of incorporation of the compounds of this invention into polymeric resins will be readily apparent to the skilled artisan. The compounds are for instance incorporated by cross-linking the hydroxyl groups of the phosphonate or hydroxymethyl substituents using cross-linking agents heretofore known. Linking through a group other than the heterocyclic base will produce a resin that may for example be useful in hydrophobic affinity chromatography.

The purine derivatives according to the invention can also be used as a modulator of α- and β-adrenergic and purinergic receptors. In a preferred embodiment, this invention thus provides a method for inhibiting or stimulating the signal transduction of adrenergic and purinergic receptors in mammals comprising administering a therapeutically effective amount of the composition of claim 1 to the mammal. The inhibiting and stimulating molecules are for example useful for treating inflammatory diseases and asthma, cardiovascular neurodegenerative and inflammatory diseases.

In another embodiment, this invention provides purine derivatives useful for treating fungal infections in humans, animal, and in plants.

The 2,6,9-trisubstituted purine derivatives of a preferred embodiment of the invention the invention having an amine substituted with a catechol or related group (1,2-dihydroxybenzene) result in the acquisition of extremely high potency against viruses, in particular DNA viruses. Moreover, surprisingly the chirally enriched or pure (S)-enantiomer is antivirally active. Heretofore, only the (R)-enantiomer was notably antivirally active, and then only against the retroviruses.

In another preferred embodiment of the invention, a method is provided for inhibiting cdks, and/or or cell proliferation and/or for inducing apoptosis in mammals, comprising administering a therapeutically effective amount of the compound according to the invention to the mammal. The cdk inhibiting molecules are useful for treating disorders, some of them involving cell proliferation, such as cancer, restenosis, rheumatoid arthritis, lupus, type I diabetes, multiple sclerosis, Alzheimer's disease, growth of parasites (animal, protists), graft rejection (host versus graft disease), graft versus host disease, and gout.

The purine derivatives of the formula I and their pharmaceutically acceptable salts for example selectively inhibit cdks, in particular the enzyme p34^{cdc2}/cyclin B kinase and related cdks (cdk2, cdk5, cdk7, cdk9, erk1, erk2). In addition to other cdc2-related kinases, this kinase controls certain steps of cell division cycles, in particular the transition from G₁ phase into the S phase and in particular the transition from the G₂ phase into the M-phase. The compounds of formula I and their pharmaceutically acceptable salts advantageously are used as antimitotic compounds, for example for the treatment of proliferative diseases, such as cancer and restenosis. In very low concentrations (micromolar and lower), they are capable of inhibiting cell cycle transitions (G₁/S, G₂/M, M-phase/metaphase) in different animal bodies and embryos.

Furthermore, the compounds are useful in treating auto-immune diseases, e.g. rheumatoid arthritis, lupus, type I diabetes, multiple sclerosis, etc.; in treating Alzheimer's disease, cardiovascular disease such as restenosis, graft rejection (host vs. graft disease), graft vs. host disease, gout, and in treating polycystic kidney disease, cancer and other proliferative diseases of which the pathogenesis involves abnormal cell proliferation.

The purine derivatives according to the invention also are potent and specific inhibitors of IκB-a kinase which prevents signal induced NF-κB activation and cytokine synthesis in vitro and in vivo. Such inhibitors inhibit synthesis of cytokines and adhesion proteins the synthesis of which is transcriptionally regulated by NF-κB. Pro-inflammatory cytokines such as IL-1, IL-6, TNF and adhesion proteins (e.g. ICAM, VCAM and selections) belong to this class of molecules and have been implicated in the pathogenesis of inflammatory diseases. Thus a potent inhibitor of IκB-α kinase is useful in the clinical management of diseases where NF-κB activation is required for disease induction.

The compounds of the invention also affect the activation and/or signal transduction of α- and β-adrenergic receptors, e.g. phosphatidyl turnover and cyclic AMP synthesis respectively. Activation of β-adrenergic receptors has an anti-inflammatory effect by decreasing the cytokine production of macrophages, astrocytes, and by preventing an increase in vascular permeability. On the other hand, a decreased β-adrenergic receptor activation is useful in diseases like multiple sclerosis and rheumatoid arthritis. The novel compounds also affect P2-purinergic receptor activation linked to phosphatidyl turnover and inhibition of activation of cyclic AMP synthesis or P1-purinergic receptor activation positively or negatively coupled to the activation of adenylate cyclase depending on the receptor subtype. Modulation of purinergic receptor signalling may be useful in cerebral ischaemia, stroke, treatments of neurodegenerative diseases (e.g. Parkinson's disease), renal failure, treatment of lung dysfunction, and in inhibition of cancer growth.

The invention further provides novel compounds activating p53, the mammal cell's own natural brake gene for stopping uncontrolled cell proliferation (cancer), thus being able to switch off the cancer. p53 as well as retinoblastoma (Rb) are two well characterised tumour suppressors whose inactivation may lead to uncontrolled cell proliferation and malignancy. Phosphorylation of these two proteins, which are involved in cell cycle regulatory mechanisms, is known to modulate their function. A potent cdk-inhibitor thus represents a good tool for treatment of cancers due to induction of wild type p53 protein in cancers expressing mutant p53.

Studies carried out on the derivatives of the invention have demonstrated, in addition, the strong effect of the purine derivativs on apoptosis of many cancer cell lines. It has been demonstrated that apoptosis can be induced at stage G₁ or G₂ and following damage of the DNA, some cells stop at stage G₁ and a p53-dependent apoptotic pathway is then induced. In other situations, cells stop at G₂/M stage in response to damage caused to the DNA, and activation of a p53-independent apoptotic path is observed. This path has proved to be particularly significant in the therapy of tumours in which less active p53 is observed. By application of the purine derivatives of the invention, p53-independent apoptosis will be stimulated in cells which have stopped at stage G₂ through damage to the DNA using agents such as mitoxantrone or cis-platinum. The cdk inhibitors of this invention can thus increase the therapeutic potential of anti-tumour agents currently used.

The compounds of this invention can furthermore be terminally incorporated into oligonucleotides. If they contain a nonphosphonyl free hydroxyl group, they optionally are incorporated internally into the sequence of the oligonucleotide. Terminally incorporated diphosphonyl compounds of this invention which contain no free hydroxyl capable of participating in chain elongation also are useful in DNA sequencing in essentially the same manner as deoxy-NTPs have been used in the past (see example 8 of U.S. Patent 5,276,143). The nucleotide analogues of the invention (when diphosphorylated) are useful as chain terminators for dideoxynucleotide-type DNA sequencing protocols, provided that the nucleotide analogue lacks a free hydroxyl group suitable for polymerase mediated chain elongation. These compounds will not have R=hydroxymethyl and do not posses a cyclic structure incorporating the phosphorus atom (although compounds having such excluded structures can be intermediates). The nucleotide analogue may be included in a kit with other reagents (such as Klenow polymerase or T4 polymerase, dNTPs, etc) needed for DNA sequencing (Otvos et al., "Nucl. Acids Res." 1987: 15: 1763-1777).

If the oligonucleotide-incorporated compound of this invention is binding-competent for its complementary sequence, i.e. if it is capable of base pairing, this nucleotide monomer will participate in hybridisation. It is not necessary, however, that the incorporated nucleotide analogue of this invention participates in hybridisation. If it is located at the terminus of the oligonucleotide, it will be useful as an immunological recognition site, or haptenic recognition site, to facilitate detection of the oligonucleotide by an antibody capable of binding the compound of this invention.

The compounds of this invention also are useful as linkers or spacers in preparation of affinity absorption matrices (as opposed to functioning as affinity moieties per se, as noted above), immobilised enzymes for process control, or immunoassay reagents. The compounds herein contain a multiplicity of functional groups that are suitable as sites for cross-linking desired substances. For example, it is conventional to link affinity reagents such as hormones, peptides, antibodies, drugs, and the like to insoluble substrates. These insolubilised bound reagents are employed in known fashion to absorb binding partners for the affinity reagents from manufactured preparations, diagnostic samples and other impure mixtures. Similarly, immobilised enzymes are used to perform catalytic conversions with easy recovery of enzyme. Bifunctional compounds are commonly used to link analytes to detectable groups in preparing diagnostic reagents.

Many functional groups present in the compounds of this invention are suitable for use in cross-linking. The R groups substituted with OH, or vinyl are exemplary suitable sites. Similarly, the amino and other reactive sites found on group R2, R6, and R9 are suitable. Suitable protection of reactive groups will be used where necessary while assembling the cross-linked reagent. In general, the compounds are used by linking through phosphonic acid or amino group to the hydroxyl or amino groups of the linking partner in the same fashion as shown, and covalently binding to the other binding partner through an R group. For example, a first binding partner such as a steroid hormone is esterified and then this conjugate is cross-linked through hydroxymethyl R to cyanogen bromide activated Sepharose, whereby an immobilised steroid is obtained. Other chemistries for conjugation are well known. See for example Maggio, "Enzyme-Immunoassay" (CRC, 1988, pp 71-135) and references cited therein.

The oligonucleotides of this invention may for example be labelled with any conventional detectable label, e.g. a fluorescent moiety such a fluorescein, radioisotopes such as ¹⁴C or ³H, stable free radicals, avidin, biotin and the like, all of which previously have been used as labels for immunoassays or diagnostic probes. The label may be present on the oligonucleotide or on the residue of an analogue of this invention. Suitable labelling methods are well known and are easily used with reactive groups such as hydroxyl, allyl and the like. A simple method is to label the compound of this invention with ³H by proton exchange. The compounds also may be biotinylated using conventional methods. See for instance U.S. Patent 5,276,143 for analogous structures. The compounds of this invention, however, also are useful directly in diagnostic probe assays without an exogenous detectable label. In one embodiment of this alternative, antibodies are raised against the compounds of the invention. Such antibodies (which in turn are labelled or used in a double antibody configuration) bind to the analogue of this invention and thereby are useful in detecting its presence as label for a protein or oligonucleotide.

The compounds of the invention are useful for treatment of microbial infections, for treatment of tumours or for other indications described below. Microbial infections treatable by the compounds of this invention include viruses, parasites, yeast and fungi, but it is believed that the compounds are most effective against viruses, which constitutes a preferred utility. Exemplary viral infections include infections caused by DNA or RNA viruses including herpesviruses (herpes simplex virus type 1 (HSV-1), HSV-2, varicella zoster virus (VZV), Epstein-Barr virus (EBV), cytomegalovirus (CMV), human herpesvirus type 6 (HHV-6), HHV-7, HHV-8, bovine herpesvirus type 1, equine herpesvirus type 1, papillomaviruses (HPV types 1-55, including carcinogenic HPV), flaviviruses (including yellow fever virus, African swine fever virus and Japanese encephalitis virus), togaviruses (including Venezuelan equine encephalomyelitis virus), influenza viruses (types A-C), retroviruses (HIV-1, HIV-2, HTLV-I, HTLV-II, SIV, FeLV, FIV, MoMSV), adenoviruses (types 1-8), poxviruses (vaccinia virus), enteroviruses (poliovirus types 1-3, Coxsackie, hepatitis A virus, and ECHO virus), gastroenteritis viruses (Norwalk viruses, rotaviruses), hantaviruses (Hantaan virus), polyomavirus, papovaviruses, rhinoviruses, parainfluenza virus types 1-4, rabies virus, respiratory synctial virus (RSV), hepatitis viruses A, B, C and E, and the like.

The antiviral activity of the individual compounds may be determined by routine assay of antiviral (or other antimicrobial) activity using enzyme inhibition assays, tissue culture assays, animal model assays and the like, as will be understood by those skilled in the art.

Protozoan parasite infections to be treated using the compounds of the invention include infections caused by for example members of the subphyla Sarcomastigophora and Sporozoa of the phylum Protozoa. More particularly, the term protozoa as used herein include genera of parasitic protozoa, which are important to man, because they either cause disease in man or in his domestic animals. These genera for the most part are classified in the superclass Mastigophora of the subphylum Sarcomastigophora and the class Telesporea of the subphylum Sporozoa in the classification according to Baker (1969). Illustrative genera of these parasitic protozoa include Histomonas, Pneumocystis, Trypanosoma, Giardia, Trichomonas, Eimeria, Isopora, Leishmania, Entamoeba, Toxoplasma and Plasmodium. Parasitic protozoans include Plasmodium falciparum, Plasmodium berghei, Plasmodium malariae, Plasmodium vivax, Leishmania braziliensis, Leishmania donovani, Trypanosoma cruzi, Trypanosoma brucei, Trypanosoma rhodesiense, Pneumocystis carinii, Entamoeba histolytica, Trichomonas vaginalis and the like (de Vries, E. et al., "Mol. Biochem. Parasitol." 1991: 47:43-50) and trypanosomes (Kaminsky et al. "J. Parasitol." 1994;80(6):1026-1030).

Compounds of the invention are also used to treat yeast or fungal infections caused by Candida glabrata, Candida troricalis, Candida albicans, and other Candida species, Cryptococcus species including Cryptococcus neoformans, Blastomyces species including Blastomyces dermatitidis, Torulopsis species including Torulopsis glabrata, Coccidioides species including Coccidioides immitis, Aspergillus species and the like.

The compounds of the invention can furthermore be (1) applied to tissue culture systems to eliminate or reduce viral spread or growth during the production of biopharmaceutical or other products (such as proteins or vaccines), (2) used to eliminate or reduce viral spread or growth in clinical samples (such as blood), and (3) used to stop growth of tissue culture cells while leaving the cells to carry on with protein production.

The compounds of the invention furthermore have been found to suppress immunostimulation. Accordingly, they can suppress metabolic activities of T-lymphocytes stimulated by diverse agents, e.g. concanavalin A. The purine derivatives of the invention will find application in the treatment of for example autoimmune diseases, e.g. arthritis, or in suppression of transplant rejection.

In another preferred embodiment the invention provides a pharmaceutical composition one or more of the purine derivatives of the invention in an admixture with one or more pharmaceutical excipients or diluents.

### PROCESSES FOR PREPARATION

The invention further provides a method to prepare the novel purine derivatives according to the invention.

The starting materials for the purine derivatives of formula I are 6-chloropurine and 2,6,-dichloropurine, prepared from hypoxanthine and hypoxanthine-1-N-oxide by chlorination with POCl_{3,} (Davoll and Blowy, J. Am. Chem. Soc. 1957, 73:2936). This starting material is also available from commercial sources (Sigma, Aldrich, Fluka, etc.). The compounds of the formula I may also be prepared from 2,6,8- and 6,8-dichloropurine prepared from uric acid by chlorination with POCl₃ (J. Am. Chem. Soc. 1958, 80:6671; J. Org. Chem. 1961, 26:447).

In one approach, the 6-substituted purines of formula I, wherein R6 substituents are as defined above, are prepared by reaction of 6-chloropurine with an appropriate amine, such as phenylglycinol, 2-, 3-, 4-hydroxybenzylamine, dihydroxybenzylamine, 4-aminoresorcinol, or 2-, 3-, 4-hydroxyaniline. 6-Chloropurine is dissolved in n-butanol and the appropriate R⁶-amine (1.5-5 eq.) and several-fold excess of triethylamine is used. After heating for several hours, the reaction mixture is cooled and the 6-substituted purine is obtained.

In another approach the 6,9-disubstituted purines of the formula I, wherein R6 and R9 substituents are as defined above, are prepared from 6-substituted purines (in DMSO, or DMF) to which powdered calcium carbonate (aproximately 3 eq.) is added, followed by R⁹⁻halogen. After several hours or days of vigorous stirring the product is isolated by means of liquid chromatography.

In yet another approach the 2,6-disubstituted purine derivatives of formula **I**, wherein **R2** and **R6** substituents are as defined above, are prepared from 2,6-dichloropurine by reacting 2,6-dichloropurine with appropriate nucleophile (phenylglycinol, 2-, 3-, 4-hydroxybenzylamine, dihydroxybenzylamine, 4-aminoresorcinol, 2-, 3-, 4-hydroxyaniline, substituted amines, mercaptoderivatives with 2 equivalents of N-methylpyrrolidone or N-ethyl-diisopropylamine, alcoholates) according to the method as described above for 6-chloropurine. Substitution of C²-Cl is then achieved by reaction with second nucleophile (5 - 30 equivalents of substituted amines, aminoalkanols; mercapto derivatives in the presence of N-methylpyrrolidinone or N-ethyl-diisopropylamine) at a temperature 160-180°C. The product is isolated by liquid chromatography or crystallized from n-BuOH or water.

In yet another approach, the 2,6,9-trisubstituted purine derivatives of formula **I**, wherein **R2, R6** and **R9** substituents are as defined above, are prepared by alkylation (K₂CO_{3,} DMSO, R⁹-halogen) of 2-chloro-6-substituted purines and subsequent reaction with R²-SH or R²-NH as described above for preparation of 2-chloro-6-substituted purine derivatives.

In another approach the 2,9-disubstituted purine derivatives of formula I, wherein R2 and R9 substituents are as defined above for a compound of formula I, are prepared from 2,6-dichloropurine in DMSO (or DMF) to which powdered potassium carbonate (5 equivalents) is added followed by R⁹-halogen (appr. 4 eq.). After one or two days of vigorous stirring, 9-alkylated-2,6-dichloropurine (detection on the basis of principal spot on TLC) is isolated by means of liquid chromatography. Selective hydrogenolysis of C⁶-Cl provides 2-chloro-9-alkylpurine (10% Pd/BaSO₄, MeOH, Et₃N, 25°C, 2 h). The last nucleophilic substitution of C²-Cl is achieved by means of reaction in excess (2-30 eqv) of the desired nucleophile (substituted amines, mercapto derivatives with 2 equivalents of N-methylpyrrolidinone or N-ethyl-diisopropylamine, alcoholates) at a temperature 140-180°C (2-48 h). 2,9-Disubstituted purines are then isolated by means of liquid chromatography.

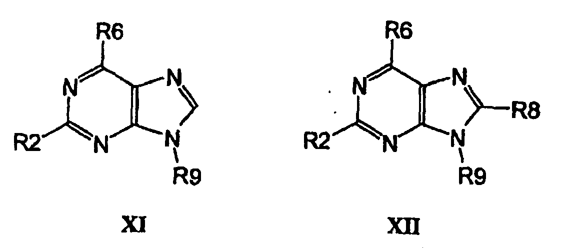

### THERAPEUTIC ADMIDTISTRATION

Suitable routes for administration of the purine derivatives according to the invention for use in the treatment of the human or animal body include for example oral, rectal, topical (including dermal, ocular, buccal and sublingual), vaginal and parenteral routes (including subcutaneous, intramuscular, intravitreous, intravenous, intradermal, intrathecal and epidural). The preferred route of administration will depend upon the condition of the patient, the toxicity of the specific derivative used and the site of infection, among other considerations known to the clinician.

In a preferred embodiment of the invention the pharmaceutical composition comprises about 1% to about 95% of one or more of the purine derivatives of the invention as the active ingredient and at least a pharmaceutically acceptable carrier or diluent, single-dose forms of administration preferably comprising about 20% to about 90% of the active ingredient and administration forms which are not single-dose preferably comprising about 5% to about 20% of the active ingredient. Unit dose forms are, for example, coated tablets, tablets, ampoules, vials, suppositories or capsules. Other forms of administration include, for example, ointments, creams, pastes, foams, tinctures, lipsticks, drops, sprays, dispersions and the like. In a preferred embodiment the purine derivatives are incorporated in capsules comprising about 0.05 g to about 1.0 g of the active ingredient.

The pharmaceutical compositions of the present invention are prepared in a manner known per se, for example by means of convectional mixing, granulating, coating, dissolving or lyophilising processes.

Preferably, solutions of the active ingredient, and in addition also suspensions or dispersions, in particular isotonic aqueous solutions, dispersions or suspensions, are used, it being possible for these to be prepared before use, as for example in the case of lyophilised compositions which comprise the active substance by itself or together with a carrier, for example mannitol. The pharmaceutical compositions can be sterilised and/or comprise excipients, for example preservatives, stabilisers, wetting agents and/or emulsifiers, solubilizing agents, salts for regulating the osmotic pressure and/or buffers, and they can be prepared in a manner known per se, for example by means of convectional dissolving or lyophilising processes. The solutions or suspensions can comprise viscosity-increasing substances, such as sodium carboxymethylcellulose, carboxymethylcellulose, dextran, polyvinylpyrrolidone or gelatin.

Suspensions-in-oil comprise, as the oily component, the vegetable, synthetic or semi-synthetic oils customary for injection purposes. Oils to be used in the invention preferably include liquid fatty acid esters which contain, as the acid component, a long-chain fatty acid having 8 - 22, in particular 12-22, carbon atoms, for example lauric acid, tridecylic acid, myristic acid, pentadecylic acid, palmitic acid, margaric acid, stearic acid, arachidonic acid, behenic acid or corresponding unsaturated acids, for example oleic acid, elaidic acid, euric acid, brasidic acid or linoleic acid, if appropriate with the addition of antioxidants, for example vitamin E, β-carotene or 3,5-di-tert-butyl-4-hydroxytoluene. The alcohol component of these fatty acid esters preferably has no more than 6 carbon atoms and is mono- or polyhydric, for example mono-, di- or trihydric alcohol, for example methanol, ethanol, propanol, butanol, or pentanol, or isomers thereof, but in particular glycol and glycerol. Suitable fatty acid esters are for example: ethyl oleate, isopropyl myristate, isopropyl palmitate, "Labrafil M 2375" (polyoxyethylene glycerol trioleate from Gattefossé, Paris), "Labrafil M 1944 CS" (unsaturated polyglycolated glycerides prepared by alcoholysis of apricot kernel oil and made of glycerides and polyethylene glycol esters; from Gattefossé, Paris), "Labrasol" (saturated polyglycolated glycerides prepared by an alcoholysis of TCM and made up of glycerides and polyethylene glycol esters; from Gattefossé, Paris) and/or "Miglyol 812" (triglyceride of saturated fatty acids of chain length C₈ to C₁₂ from Hüls AG, Germany), and in particular vegetable oils, such as cottonseed oil, almond oil, olive oil, castor oil, sesame oil, soybean oil and, in particular, groundnut oil.

The preparation of the injection compositions is carried out in customary manner under sterile conditions, as are bottling, for example in ampoules or vials, and closing of the containers.

Pharmaceutical compositions for oral use can for example be obtained by combining the active ingredient with one or more solid carriers, if appropriate granulating the resulting mixture, and, if desired, processing the mixture or granules to tablets or coated tablet cores, if appropriate by addition of additional excipients. Suitable carriers are, in particular, fillers, such as sugars, for example lactose, sucrose, mannitol or sorbitol, cellulose preparations and/or calcium phosphates, for example tricalcium diphosphate, or calcium hydrogen phosphate, and furthermore binders, such as starches, for example maize, wheat, rice or potato starch, methylcellulose, hydroxypropylmethylcellulose, sodium carboxymethylcellulose and/or polyvinylpyrrolidine, and/or, if desired, desintegrators, such as the above mentioned starches, and furthermore carboxymethyl-starch, cross-linked polyvinylpyrrolidone, alginic acid or a salt thereof, such as sodium alginate. Additional excipients are, in particular, flow regulators and lubricants, for example salicylic acid, talc, stearic acid or salts thereof, such as magnesium stearate or calcium stearate, and/or polyethylene glycol, or derivatives thereof.

Coated tablet cores can be provided with suitable coatings which, if appropriate, are resistant to gastric juice, the coatings used being, inter alia, concentrated sugar solutions, which, if appropriate, comprise gum arabic, talc, polyvinylpyrrolidine, polyethylene glycol and/or titanium dioxide, coating solutions in suitable organic solvents or solvent mixtures or, for the preparation of coatings which are resistant to gastric juice, solutions of suitable cellulose preparations, such as acetylcellulose phthalate or hydroxypropylmethylcellulose phthalate. Dyes or pigments can be admixed to the tablets or coated tablet coatings, for example for identification or characterisation of different doses of the active ingredient.

Pharmaceutical compositions, which can be used orally, are also hard capsules of gelatin and soft, closed capsules of gelatin and a plasticiser, such as glycerol or sorbitol. The hard capsules can contain the active ingredient in the form of granules, mixed for example with fillers, such as maize starch, binders and/or lubricants, such as talc or magnesium stearate, and stabilisers if appropriate. In soft capsules, the active ingredient is preferably dissolved or suspended in suitable liquid excipients, such as greasy oils, paraffin oil or liquid polyethylene glycols or fatty acid esters of ethylene glycol or propylene glycol, it being likewise possible to add stabilisers and detergents, for example of the polyethylene sorbitan fatty acid ester type.

Other oral forms of administration are, for example, syrups prepared in the customary manner, which comprise the active ingredient, for example, in suspended form and in a concentration of about 5% to 20%, preferably about 10% or in a similar concentration which results in a suitable individual dose, for example, when 5 or 10 ml are measured out. Other forms are, for example, also pulverulent or liquid concentrates for preparing shakes, for example in milk. Such concentrates can also be packed in unit dose quantities.

Pharmaceutical compositions, which can be used rectally, are, for example, suppositories that comprise a combination of the active ingredient with a suppository base. Suitable suppository bases are, for example, naturally occurring or synthetic triglycerides, paraffin hydrocarbons, polyethylene glycols or higher alkanols.

Compositions which are suitable for parenteral administration are aqueous solutions of an active ingredient in water-soluble form, for example of water-soluble salt, or aqueous injection suspensions, which may comprise viscosity-increasing substances, for example sodium carboxymethylcellulose, sorbitol and/or dextran, and if appropriate stabilisers. The active ingredient can also be present in the form of a lyophilisate, if appropriate together with excipients, and be dissolved prior to parenteral administration by addition of suitable solvents. Solutions such as are used, for example, for parenteral administration can also be used as infusion solutions. Preferred preservatives are, for example antioxidants, such as ascorbic acid, or microbicides, such as sorbic or benzoic acid.

Ointments are oil-in-water emulsions, which comprise not more than 70%, but preferably 20 - 50% of water or aqueous phase. The fatty phase may for example consist of hydrocarbons, for example vaseline, paraffin oil or hard paraffin's, which preferably comprise suitable hydroxy compounds, such as fatty alcohol's or esters thereof, for example cetyl alcohol or wool wax alcohols, such as wool wax, to improve the water-binding capacity. Emulsifiers are corresponding lipophilic substances, such as sorbitan fatty acid esters (Spans), for example sorbitan oleate and/or sorbitan isostearate. Additives to the aqueous phase are, for example, humectants, such as polyalcohols, for example glycerol, propylene glycol, sorbitol and/or polyethylene glycol, or preservatives and odoriferous substances.

Fatty ointments are anhydrous and may comprise, as the base, in particular, hydrocarbons, for example paraffin, vaseline or paraffin oil, and furthermore naturally occurring or semi-synthetic fats, for example hydrogenated coconut-fatty acid triglycerides, or, preferably, hydrogenated oils, for example hydrogenated groundnut or castor oil, and furthermore fatty acid partial esters of glycerol, for example glycerol mono- and/or distearate, and for example, the fatty alcohols. They also may contain emulsifiers and/or additives mentioned in connection with the ointments which increase uptake of water.

Creams are oil-in-water emulsions, which comprise more than 50% of water. Oily bases used are, in particular, fatty alcohols, for example lauryl, cetyl or stearyl alcohols, fatty acids, for example palmitic or stearic acid, liquid to solid waxes, for example isopropyl myristate, wool wax or beeswax, and/or hydrocarbons, for example vaseline (petrolatum) or paraffin oil. Emulsifiers are surface-active substances with predominantly hydrophilic properties, such as corresponding non-ionic emulsifiers, for example fatty acid esters of polyalcohols or ethyleneoxy adducts thereof, such as polyglyceric acid fatty acid esters or polyethylene sorbitan fatty esters (Tweens), and furthermore polyoxyethylene fatty alcohol ethers or polyoxyethylene fatty acid esters, or corresponding ionic emulsifiers, such as alkali metal salts of fatty alcohol sulfates, for example sodium lauryl sulfate, sodium cetyl sulfate or sodium stearyl sulfate, which are usually used in the presence of fatty alcohols, for example cetyl stearyl alcohol or stearyl alcohol. Additives to the aqueous phase are, inter alia, agents which prevent the creams from drying out, for example polyalcohols, such as glycerol, sorbitol, propylene glycol and/or polyethylene glycols, and furthermore preservatives and odoriferous substances.

Pastes are creams and ointments having secretion-absorbing powder constituents, such as metal oxides, for example titanium oxide or zinc oxide, and furthermore talc and/or aluminium silicates, which have the task of binding the moisture or secretions present.

Foams may be administered from pressurised containers and may be liquid oil-in-water emulsions present in aerosol foam. As the propellant gases, halogenated hydrocarbons, such as polyhalogenated alkanes, for example dichlorofluoromethane and dichlorotetrafluoroethane, or, preferably, non-halogenated gaseous hydrocarbons, air, N₂O, or carbon dioxide are used. The oily phases and the additives used are, inter alia, those mentioned above for ointments and creams.

Tinctures and solutions usually comprise an aqueous-ethanolic base to which, for example, humectants for reducing evaporation, such as polyalcohols, for example glycerol, glycols and/or polyethylene glycol, and re-oiling substances, such as fatty acid esters with lower polyethylene glycols, i.e. lipophilic substances soluble in the aqueous mixture to substitute the fatty substances removed from the skin with the ethanol, and, if necessary, other excipients and additives, are admixed.

The pharmaceutical compositions according to the invention may also comprise veterinary compositions comprising at least one active ingredient as above defined together with a veterinary carrier therefor. Veterinary carriers are materials for administering the composition and may be solid, liquid or gaseous materials, which are inert or acceptable in the veterinary art and are compatible with the active ingredient. These veterinary compositions may be administered orally, parenterally or by any other desired route.

The invention also provides methods for treatment of several diseases, such as those disease states mentioned above. The compounds can be administered prophylactically or therapeutically as such or in the form of pharmaceutical compositions, preferably in an amount, which is effective against the diseases mentioned. With a warm-blooded animal, for example a human, requiring such treatment, the compounds are used, in particular, in the form of pharmaceutical composition.

The invention is further illustrated by the following Examples and figures, which only serve to illustrate the invention without limiting the scope thereof.
Figure 1 shows a comparison between normal PHA-stimulated lymphocytes and hematopoietic cell lines, demonstrating that lymphocytes are more sensitive to some of the novel compounds than cell lines, with the exception of P23, that was significantly more effective on KG1 than on normal PBMC.
Figure 2 shows the results of microscopic examination of KG1 cells incubated with P23. Viable, apoptotic, necrotic, and secondarily necrotic cells were scored after 6, 12, 24, 48 and 72 hours of exposure to the compounds of the invention.

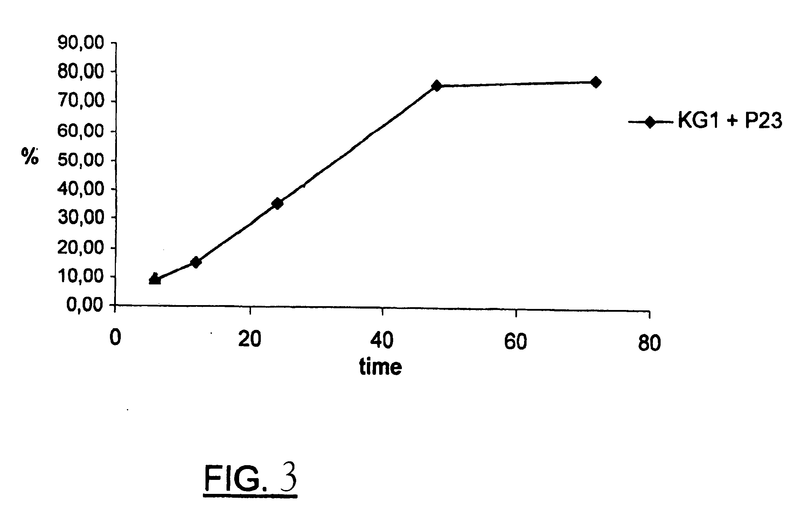

Figure 3 is a graph showing the percentage of cells with DNA fragmentation (apoptosis) as detected by the TUNEL technique in cell line KG1 after incubation with P23 over a period of 72 hours.

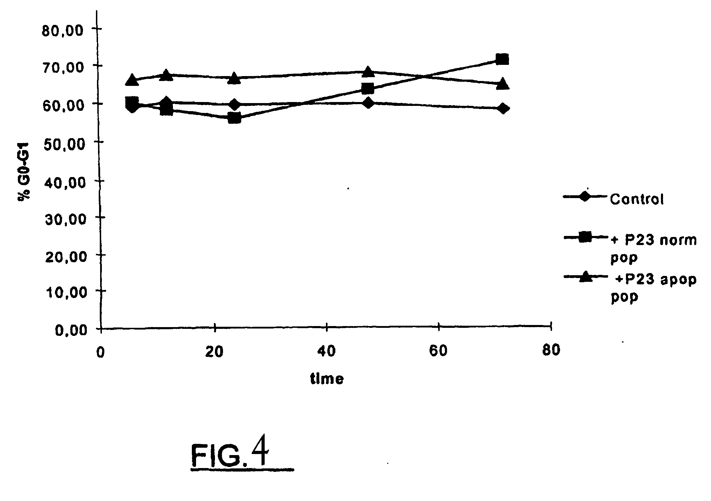

Figure 4 shows graphs demonstrating the percentage of KG1 cells in G₀-G₁ phase as detected by DNA staining with ethidium bromide (EB) for P23. TUNEL in combination with EB shows the percentage of apoptotic (apop pop) and non-apoptotic (norm pop) cells in G₀-G₁.

### EXAMPLES

### EXAMPLE 1:

### 6-[(RS)-(1-Phenyl-2-hydroxyethyl)amino]purine

6-Chloropurine (3 mmol, 0.47g), (RS)-2-phenylglycinol (5 mmol, 0.7g), and triethylamine (2 mL) were heated with stirring in 10 mL of 1-butanol (125°C, 3h). After cooling, the volatile components were evaporated. The product was obtained by crystallization from acetic acid-ethyl acetate. Recrystallization from hot acetic acid gave the desired product; yield 82%, mp 130-138°C. MS(Waters/Micromas ZMD detector, direct inlet, MeOH +AcOH solution, ESI 20 eV): 256.4 (100%), [M+H]⁺⁻. PTIR(Nicolet 205, KBr, cm⁻¹) : 1695, 1623, 1607, 1587, 1411, 1368, 1317, 1291.

### 2-Chloro-6-[(S)-(1-phenyl-2-hydroxyethyl)amino]purine

2,6-Dichloropurine (1.66 mmol, 0.31g), (S)-2-phenylglycinol (2mmol, 0.27g) and triethylamine (0.3 mL) were heated in 5 mL of 1-butanol (120°C, 1h). After evaporation of the volatile components the product was isolated by means of column chromatography (silica gel, CHCl₃/MeOH/AcOH; 60/2.8/1.2). Crystallization from MeOH-Et₂O gave 0.33g of the product; yield 86%; mp 205-210°C; [α]_{D}=+21.9 (MeOH, c=0.22).

Other purine derivatives which were prepared by the method of Example 1 are listed in table 1.

**Table 1: Purine derivatives prepared by the method of Example 1**

| PURINE SUBSTITUENT | | |
|---|---|---|
| C2 | N6 | C8 |
| | [N-(3,4-dihydroxybenzyl-N-methyl]amino | |
| | 3,4-dihydroxybenzylamino | |
| | [(R,S)-(1-phenyl-2-hydroxyethyl)amino] | |
| Chloro | [N-(3,4-dihydroxybenzyl-N-methyl]amino | |
| Chloro | 3,4-dihydroxybenzylamino | |
| | [(R,S)-(1-phenyl-2-hydroxyethyl)amino] | |
| | [(R,S)-(1-phenyl-2-hydroxyethyl)amino] | |
| Chloro | [(R,S)-(1-phenyl-2-hydroxyethyl)amino] | |

### EXAMPLE 2:

### 2-chloro-9-isopropyl-6-[(S)-(1-phenyl-2-hydroxyethyl)amino]purine

2-chloro-6-[(S)-(1-phenyl-2-hydroxyethyl)amino]purine (0.6 mmol, 0.17g), powdered potassium carbonate (1.4 mmol, 0.2g) and isopropylbromide (4.2 mmol, 0.4 mL) were vigorously stirred in dry DMF (2 mL) for 24 hours. 2-bromopropane (0.4 mL) was added and the reaction was continued for another 48 hours. After evaporation of the volatile components the residue was partitioned between water and ethyl acetate. The organic layer was dried over sodium sulphate. The product was crystallized from diethylether (0.167 g). Yield 84%, mp 150-152°C, [α]_{D}=+19.2 (c=0.22; CHCl₃). MS EI(Jeol JMS-D100, 80 eV, 300υA, 200°C, direct inlet): 331.1183 (M⁺⁻; C₁₅H₁₈N₅OCl, theor. 331.1200; 0.3), 301(48), 300.1013 (C₁₅H₁₅N₅Cl, theor. 300.1016; 89), 258(100),222(11), 195 (6), 155(6), 153(9), 134(6), 119(18), 106(11), 91(9), 77(11).

### 2- (S)-(2-hydroxypropyl)amino-9-isopropyl-6-[(S)-(1-phenyl-2-hydroxyethyl)amino] purine

2-chloro-9-isopropyl-6-[(S)-(1-phenyl-2-hydroxyethyl)amino]purine (0.28 mmol, 94 mg) and (S)-2-hydroxypropylamine (2.8 mmol,0.22 mL) were heated (sealed ampoule, 160°C, 3h) in diglyme (0.5 mL). After evaporation of the solvent and an excess of amine, the product was purified by column chromatography (silica gel, 3% MeOH in CHCl₃). Crystallization from CHCl₃-Et₂O afforded 2-(S)-(2-hydroxyethyl)amino-9-isopropyl-6-[(RS)-(1-phenyl-2-hydroxyethyl)amino]purine (75mg); yield 72%; mp 110-112°C; [α]_{D} +58.6 (c=0.2, CHCl₃). MS EI(Jeol JMS-D100, 80 eV, 300uA, 200°C, direct inlet): 370.2129(M⁺⁻; C₁₉H₂₆N₆O, theor. 370.2117; 15), 352(8), 340(49), 339(100), 325(8), 321(18), 297(19), 296(23), 295(26), 282(11), 279(41), 205(40), 163(24), 134(17), 106(22), 91(15), 41(12). ¹H NMR(200 MHz, CDCl₃) : 1.20 d(3H, J=6.4, CH₃CHOH), 1.52 d(6H, J=6.5, (CH₃)₂CH), 1.8 bs(1H, exch), 3.32 m(2H, CH₂NH), 4.00 d(2H, J=5.0, CH₂OH), 4.00 m (1H, CHOH), 4.59 sept (1H, J=6.5, CH(CH₃)₂), 5.16 t(1H, CHPh), 5.3 bs(1H, exch), 6.50 bs(1H, exch), 7.22-7.40 m(5H, ph), 7.50 s(1H, HC⁸).

### 2-(R)(2-hydroxypropyl)amino-9-isopropyl-6-[(S)-(1-phenyl-2-hydroxyethyl)amino]purine

This purine derivative was prepared in the same manner as the previous isomer with the exception that the (R)-2-hydroxypropylamine was used instead of (S)-2-hydroxypropylamine; yield 70%; mp 109-112°C; [α]_{D}=+43.6 (c=0.15, CHCl₃). MS EI(Jeol JMS-D100, 80 eV, 300uA, 200°C, direct inlet): 370.2129 (M⁺⁻; C₁₉H₂₆N₆O, teor. 370.2117; 15), 352(5), 340(49), 339(100), 325(8), 321(18), 297(19), 296(23), 295(26), 282(11), 279(41), 205(40), 163(25), 134(17), 106(22), 91(15), 41(12). ¹H NMR(200 MHz, CDCl₃): 1.20 d(3H, J=6.4, CH₃CHOH), 1.53 d(6H, J=6.5, (CH₃)₂CH), 1.8 bs(1H, exch), 3.32 m(2H, CH₂NH), 4.01 d(2H, J=5.0, CH₂OH), 4.00 m (1H, CHOH), 4.59 sept (1H, J=6.5, CH(CH₃)₂), 5.16 t(1H, CHPh), 5.3 bs(1H, exch), 6.50 bs(1H, exch), 7.22-7.40 m(5H, ph), 7.50 s(1H, HC⁸).

### 2-hexylamino-9-isopropyl-6-[(S)-(1-phenyl-2-hydroxyethyl)amino]purine

2-chloro-9-isopropyl-6-[(S)-(1-phenyl-2-hydroxyethyl) amino] purine (0.3 mmol, 100 mg) was heated in n-hexylamine (3 mL) (sealed ampoule, 160°C, 3h). Excess of the amine was evaporated and the product was purified by column chromatography (silica gel, stepwise 0, 1, 2, % MeOH in CHCl₃). The syrup-like product (110 mg, 92%) crystallized spontaneously after several weeks; mp was too low for measuring. MS EI(Jeol JMS-D100, 80 eV, 300υA, 200°C, direct inlet): 396 (M⁺⁻; 12), 378 (17), 377(10), 366(56), 365(100), 323(41), 307(10), 301(7), 295 (10), 251 (8), 239 (14) , 205(12), 163 (9), 134 (16), 106(23), H NMR(400 MHz, CDCl₃): 0.907 t(3H, J=6.9, CH₃CH₂), 1.287-1.1420 m(4H, (CH₂)₂), 1.521-1.65 m (10H, (CH₂)₂ + (CH₃)₂CH), 3.31-3.42 m(2H, CH₂N), 3.955-4.08 m(2H,CH₂OH), 4.635 sept(1H, J=6.8, CH(CH₃)₂), 4.74 bs(1H, NHCH₂), 5.315 bs(1H, CHCH₂OH), 7.28-7.42 m (5H, Ph), 7.496(1H, HC⁸). The 2D-COSY spectra were used for the structural assignment of proton signals.

Table 2 lists the purine derivatives according to the invention that were prepared by the method of Example 2.

**Table 2: Purine derivatives prepared by the method of Example 2**

| PURINE SUBSTITUENT | | |
|---|---|---|
| C2 | N6 | N9 |
| | [(R,S)-(1-phenyl-2-hydroxyethyl)amino] | Isopropyl |
| Chloro | [(R,S)-(1-phenyl-2-hydroxyethyl)amino] | Isopropyl |
| 2-hydroxypropylamino | [(R,S)-(1-phenyl-2-hydroxyethyl)amino] | Isopropyl |
| 3-hydroxypropylamino | [(R,S)-(1-phenyl-2-hydroxyethyl)amino] | Isopropyl |
| (R)-1-(hydroxyrnethyl)-propylasnino | [(R,S)-(1-phenyl-2-hydroxyethyl)amino] | Isopropyl |
| [bis-(2-hydroxyethyl)]-amino | [(R,S)-(1-phenyl-2-hydroxyethyl)amino] | Isopropyl |
| 2-(1R-isopropyl-2-hydroxyethylamino) | [(R,S)-(1-phenyl-2-hydroxyethyl)amino] | Isopropyl |
| | [N-(3,4-dihydroxybenzyl-N-methyl]amino | Isopropyl |
| Chloro | [N-(3,4-dihydroxybenzyl-N-methyl]amino | Isopropyl |
| 3-hydroxypropylamino | [N-(3,4-dihydroxybenzyl-N-methyl]amino | Isopropyl |
| (R)-1-(hydroxymethyl) propylamino | [N-(3,4-dihydroxybenzyl-N-methyl]amino | Isopropyl |
| [bis-(2-hydroxyethyl)]-amino | [N-(3,4-dihydroxybenzyl-N-methyl]amino | Isopropyl |
| 2-(1R-isopropyl-2-hydroxyethylamino) | [N-(3,4-dihydroxybenzyl-N-methyl]amino | Isopropyl |
| | 3,4-dihydroxybenzylamino | Isopropyl |
| Chloro | 3,4-dihydroxybenzylamino | Isopropyl |
| 3-hydroxypmpylamino | 3,4-dihydroxybenzylamino | Isopropyl |
| (R)-1-(hydroxymethyl) propylamino | 3,4-dihydroxybenzylamino | Isopropyl |
| [bis-(2-hydroxyethyl)]-amino | 3,4-dihydroxybenzylamino | Isopropyl |
| 2-(1R-isopropyl-2-hydroxyethylamino) | 3,4-dihydroxybenzylamino | Isopropyl |
| | [1-(3,4-dihydroxyphenyl)ethyl]amino | Isopropyl |
| Chloro | [1-(3,4-dihydroxyphenyl)ethyl]amino | Isopropyl |
| 2-hydroxyethylamino | [1-(3,4-dihydiroxyphenyl)ethyl]amino | Isopropyl |
| 3-hydroxypropylamino | [1-(3,4-dihydroxyphenyl)ethyl]amino | Isopropyl |
| (R)-1-(hydroxymethyl) propylamino | [1-(3,4-dihydroxyphenyl)ethyl]amino | Isopropyl |
| [bis-(2-hydroxyethyl)]-amino | [1-(3,4-dihydroxyphenyl)ethyl]amino | Isopropyl |
| 2-(1R-isopropyl-2-hydroxyethylamino) | [1-(3,4-dihydroxyphenyl)ethyl]amino | Isopropyl |
| 2-(1R-isopropyl-2-hydroxyethylamino) | [1-(3,4-dihydroxyphenyl)ethyl]amino | Methyl |
| 2-(1R-isopropyl-2-hydroxyethylamino) | [1-(3,4-dihydroxyphenyl)ethyl]amino | Ethyl |
| 2-(1R-isopropyl-2-hydroxyethylamino) | [1-(3,4-dihydroxyphenyl)ethyl]amino | 2-hydroxyethyl |
| | [N-(2-(3,4-dihydroxyphenyl)ethyl)-N-methyl]amino | Isopropyl |
| Chloro | [N-(2-(3,4-dihydroxyphenyl)ethyl)-N-methyl]amino | Isopropyl |
| 3-hydroxypropylamino | [N-(2-(3,4-dihydroxyphenyl)ethyl)-N-methyl]amino | Isopropyl |
| (R)-1-(hydroxymethyl) propylamino | [N-(2-(3,4-dihydroxyphenyl)ethyl)-N-methyl]amino | Isopropyl |
| [bis-(2-hydroxyethyl)]-amino | [N-(2-(3,4-dihydroxyphenyl)ethyl)-N-methyl]amino | Isopropyl |
| 2-(1R-isopropyl-2-hydroxyethylamino) | [N-(2-(3,4-dihydroxyphenyl)ethyl)-N-methyl]amino | Isopropyl |
| 2-(1R-isopropyl-2-hydroxyethylamino) | [N-(2-(3,4-dihydroxyphenyl)ethyl)-N-methyl]amino | Methyl |
| 2-(1R-isopropyl-2-hydroxyethylamino) | [N-(2-(3,4-dihydroxyphenyl)ethyl)-N-methyl]amino | Ethyl |
| 2-(1R-isopropyl-2-hydroxyethylamino) | [N-(2-(3,4-dihydroxyphenyl)ethyl)-N-methyl]amino | 2-hydroxyethyl |

### EXAMPLE 3

### cdk inhibition assays

Cyclin-dependent kinases (p34^{cdc2}, p33^{cdk2}, p33^{cdk4}) and cyclins (cyclin B, E and D1) are produced in Sf9 insect cells coinfected with appropriate baculoviral constructs. The cells are harvested 68-72 hrs post infection in lysis buffer for 30 min on ice and the soluble fraction is recovered by centrifugation at 14.000 g for 10 min. The protein extract is stored at -80 °C.

Rb-GST is produced using an E. coli expression system, containing a sequence encoding the C terminus of retinoblastoma protein (aminoacids 773-928), which is known to be phosphorylated by p33^{cdk4} kinase. The fusion protein is purified on glutathione-agarose beads. Lysis buffer: 50mM Tris pH 7.4, 150mM NaCl, 5mM EDTA, 20mM NaF, 1% Tween, 1mM DTT, 0.1mM PMSF, leupeptine, aprotonine.

### Enzyme inhibition assays

To carry out experiments on kinetics under linear conditions, the final point test system for kinase activity measurement is used. The kinase is added to the reaction mixture in such a way that linear activity is obtained with respect to the concentration of enzyme and with respect to time.

The p34^{cdc2} and p33^{cdk2} kinase inhibition assays involve the use of 1mg/ml histone H1 (Sigma, type III-S) in the presence of 15µM [γ-³²P]ATP (500-100 cpm/pmol) (Amersham) in a final volume of 20 µl. Inhibition of p33^{cdk4} kinase is determined with Rb-GST (0.2mg/ml) as the substrate. Kinase activity is determined at 30 °C in kinase buffer.

Tested compounds are usually dissolved to 100mM solutions in DMSO, the final concentration of DMSO in reaction mixture never exceeds 1%. The controls contain suitable dilutions of DMSO. After 10 min, the addition of 3x SDS sample buffer stops the incubations. Phosphorylated proteins are separated electrophoretically using 12.5% SDS polyacrylamide gel. The measurement of kinase activity is done using digital image analysis. The kinase activity is expressed as a percentage of maximum activity, the apparent inhibition constants are determined by graphic analysis. Kinase buffer: 50mM Hepes pH 7.4, 10mM MgCl₂, 5mM EGTA, 10 mM 2-glycerolphosphate, 1mM NaF, 1mM DTT.

Table 3 shows the results of the inhibitory activity of the purine derivatives according to the invention against CDC2 and IκB-α. The 2,6,9-trisubstituted purine derivatives showed marked inhibitory activity in in vitro kinase assays. Modification of 2,6,9-trisubstituted purines by a catechol-like substituent at R6 leads to an increase in cdk inhibitory activity of the tested compounds.

**Table 3: Kinase-inhibitory activity of 2,6,9-trisubstituted purine derivatives according to the invention**

| SUBSTITUENT | | | CDC2 | IκB-α |
|---|---|---|---|---|
| C2 | N6 | N9 | IC₅₀ (µM) | IC₅₀ (µM) |
| (R)-1-(hydroxymethyl) propylamino | 3,4-dihydroxybenzylamino | Isopropyl | 0.32 | 1.25 |
| Hexylamino | [N-(2-(3,4-dihydroxyphenyl)ethyl)-N-methyl]amino | Isopropyl | 2.6 | |
| (R,S)-2-hydroxypropyl amino | [(R,S)-(1-phenyl-2-hydroxyethyl)amino] | Isopropyl | 0.6 | |
| (R)-1-(hydroxymethyl) propylamino | [(R,S)-(1-phenyl-2-hydroxyethyl)amino] | Isopropyl | 0.67 | 2.15 |
| (R)-1-(hydroxymethyl) propylamino | [N-(2-(3,4-dihydroxyphenyl)ethyl)-N-methyl]amino | Isopropyl | 0.95 | 3.20 |
| 2-(1R-isopropyl-2-hydroxyethylamino) | 3,4-dihydroxybenzylamino | Isopropyl | 250 nM | 320 nM |
| 2-(1R-isopropyl-2-hydroxyethylamino) | [(R,S)-(1-phenyl-2-hydroxyethyl)amino] | Isopropyl | 540 nM | 670 nM |
| 2-(1R-isopropyl-2-hydroxyethylamino) | [N-(2-(3,4-dihydroxyfenyl)ethyl)-N-methyl]amino | Isopropyl | 390 nM | 460 nM |

### EXAMPLE 4

### Modulation of the activity or signal transduction of β-adrenergic/purinergic receptors

Rat C6 glioma (ATCC N° CCL107) was cultivated in monolayer in serum-free chemically defined medium containing Ham's F10/minimal essential medium (1:1 vol/vol), 2mM L-glutamine, 1% (vol/vol) minimal essential medium vitamins (100x), 1% (vol/vol) minimal essential medium nonessential amino acids (100x), 100U/ml penicillin, 100µg/ml streptomycin and 30nM sodium selenite. Incubation was at 37°C in a humidified atmosphere. Assays were performed in the logaritmic growth phase at a density of 2.5x10⁵ cells/cm². Intracellular cAMP synthesis was induced by addition of 5µM (-) isoproterenol. After 30 min incubation at 37°C the medium was removed and the cellular amount of cAMP determined using the cAMP-enzyme immunoassay kit of Amersham. The IC₅₀ is determined from a dose-response curve in duplicate. The effect of seven purine-analogs was measured after simultaneous addition with isoproterenol.

**Table 4: Modulation of the activity of β-adrenergic receptors by substituted purine derivatives**

| Analog | C2 | N6 | N9 | Effect | I₅₀(µM) |
|---|---|---|---|---|---|
| P23 | Hexylamino | [(R,S)-(1-phenyl-2-hydroxyethyl)amino] | Isopropyl | inhibition | 15±2 |
| P19 (50µM) | (R)-(1-hydroxymethyl) propylamino | 4-hydroxybenzyl amino | Isopropyl | 1.8- fold activation | |
| P29 (50µM) | (R)-(1-hydroxymethyl) propylamino | 3-hydroxybenzyl amino | Isopropyl | 1.7-fold activation | |
| P38 | (S)-(1-hydroxymethyl) propylamino | (R) - hydroxy-1-phenylethylamino | Isopropyl | inactive | |
| P39 | 2-hydroxypropylamino | (R) - hydroxy-1-phenylethylamino | Isopropyl | inactive | |

As P2Y₁-like and A2 purinergic receptors, negatively and positively coupled to adenylate cyclase respectively, are present on rat C6 glioma the modulation of the synthesis of cAMP may be due to inhibition of the activation of β-adrenergic receptors by isoproterenol or to activation of purinergic receptors.

### EXAMPLE 5

### Effect of the novel compounds on the proliferation of hematopoietic cells

### Cell separation and cell cultures:

Cell lines: Human leukemic cell lines were obtained from the American Type Culture Collection (ATCC, Rockville, MD, USA). They were cultured in Iscove's Modified Dulbecco's Medium (IMDM) supplemented with 10% heat inactivated fetal calf serum (FCS), 200 U/ml penicillin, 200 µg/ml streptomycin and 1 µg/ml amphotericin B. Cells were cultured in a 5 % CO₂-95% air fully humidified incubator. For effects on clonogenic output, 1000 cells/well were plated in duplicate in methylcellulose (0.9%), supplemented with 20 % FCS and cultured for 14 days.

### Peripheral blood mononuclear cells (PBMC):

Human peripheral blood mononuclear cells were isolated by density gradient (Ficoll-Hypaque) (LSM, ICN Biomedicals Inc.). PBMC were stimulated with 5 µg/ml phytohemaglutinin A (PHA) (Sigma) during 24-48 hours in IMDM supplemented with 10% FCS (IMDM/10% FCS) at 37°C. After washing off the PHA, PBMC were incubated with interleukin-2 (IL-2) (10 U/ml) (Genzyme).

### Adult bone marrow (ABM) cells:

Bone marrow samples were obtained by sternal puncture from hematologically normal donors undergoing cardiac surgery, after obtaining informed consent. Cells were collected in IMDM supplemented with 10% FCS and 100 U/ml heparin and separated by density gradient as mentioned for PBMC. After washing, cells were resuspended in IKDM/10% FCS and were sorted on a FACStar (Becton Dickinson, Erembodegem, Belgium).

### CD34⁺ cell sorting:

ABM cells (10⁷ cells/ml) were incubated with 43A1 supernatant in a 1/10 dilution for 15 minutes at 4 °C. The supernatant of the 43A1 hybridoma (immunoglobulin (Ig)G3) was kindly donated by Dr. H. J. Bühring (University of Tübingen, Germany) and was used as a source of anti-CD34 antibodies. Then cells were washed twice in IMDM and incubated with FITC-conjugated rabbit anti-mouse Ig (1/50 dilution) for 15 minutes at 4°C. After washing twice in IMDM, CD34⁺ were sorted on a FACStarPlus cell sorter equipped with an water-cooled argon ion laser (INNOVA Enterprise Ion Laser) with multiple wave lengths including UV (488 nm).

### Myeloid Colony-forming unit (CFU) assays:

Direct myeloid colony formation of CD34⁺ cells was assessed in a CFU assay. These assays were initiated with 500 cells per well and plated in duplicate in methylcellulose (0.9%) supplemented with 20 % FCS, 1% bovine serum albumin (BSA), 10⁻⁵ M mercaptoethanol and 10 vol.% of conditioned medium of the 5637 bladder carcinoma cell line (containing G-CSF and GM-CSF), 2 U/ml erythropoietin and 30 U/ml Interleukin 3 (IL-3). After 14 days of culture at 37 °C in 7.5 % O₂ and 5% CO₂ in a fully humidified incubator, these cultures were scored with the microscope for colony formation. The following colony types were scored : myeloid colonies : macrophage (CFU-M), granulocyte (CFU-G), and granulocyte-macrophage (CFU-GM); erythroid colonies (BFU-E (burst-forming units, erythroid) and CFU-E); and mixed erytroid-myeloid colonies (CFU-Mix).

### CD34⁺CD38⁻ cell sorting:

ABM cells (10⁷ cells/ml) were incubated with 43A1 hybridoma supernatant at a 1/10 dilution for 20 minutes at 4 °C. After washing twice in IMDM, the cells were incubated with FITC-conjugated rabbit anti-mouse IgG (1/40 dilution) for 20 min at 4°C. After washing twice, cells were incubated for 10 minutes with 5 µg mouse Ig and for 15 minutes with anti-CD38-PE (20 µl/10⁶cells). After washing twice in IMDM, the cells were sorted on a FACStarPlus cell sorter equipped with an water-cooled argon ion laser (INNOVA Enterprise Ion Laser) with multiple wave length outputs including UV (488 nm). Cells with low-to-medium forward and low side scatter, highly positive green (CD34) fluorescence, and an orange (CD38) fluorescence signal lower than the mean fluorescence of cells labeled with an irrelevant isotype-matched control antibody + 2 standard deviations were retained as CD34⁺CD38⁻ cells and used in pre-CFU assays.

### Pre-CFU:

Pre-CFU assays were initiated by performing liquid cultures of CD34⁺CD38⁻ cells in duplicate in 96-well flat-bottomed plates in IMDM/10%FCS, 1 % bovine serum albumin (BSA), 100 U/ml IL-1, 200 U/ml IL-6, 30 U/ml IL-3 and 100 ng/ml stem cell factor (SCF). Pre-CFU cultures were initiated with 500 CD34⁺CD38⁻ cells/well (200 µl). After 14 days of culture at 37 °C in 7.5 % O2 and 5% CO2 in a fully humidified incubator, the number of cells in each well was counted. Subsequently, the cells were harvested, washed three times in IMDM/10% PCS, and plated in duplicate at 500 cells/well (1000 µl) in secondary methylcellulose CFU cultures as described for CFU assays.

### Effect of new compounds on cell proliferation

Cells were plated at 10000 per well in 200 µl IMDM medium and incubated with 0-50 µM of the compounds of the invention (Table 5), by addition of the drugs directly to the culture medium and incubated at 37°C for 96 hours. Absolute cell number was determined by addition of a known concentration of Fluoresbrite microspheres (FITC) (Polysciences, Inc.). The absolute number of cells per well was calculated as: {(total number of beads added per well)/(number of beads measured)} X (number of measured cells in the gate of interest). All analyses were performed with a FACScan (BD), using CELLQuest software (Becton Dickinson).

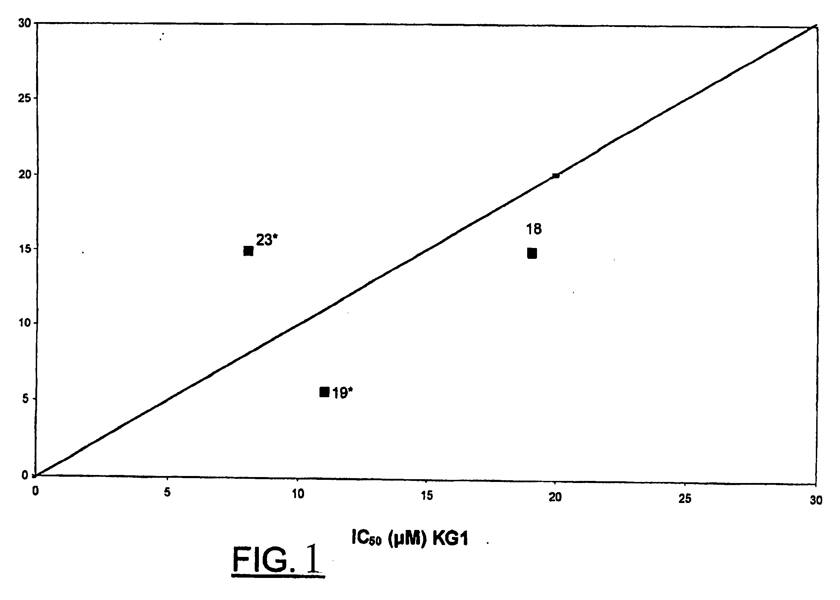

The response of the myeloid leukemia KG1 and T-lymphocyte leukemia Molt3 cell lines to the cytostatic effect of the novel compounds was determined using the above-mentioned standardized bead suspension that was used to determine the absolute cell number by flow cytometric (FCM) measurement. Cells were grown in the presence of increasing concentrations of the compounds of the invention. After 96 hours of culture the concentration at which cell growth was inhibited by 50% - the 50% inhibitory concentration or IC₅₀ - was calculated from dose-response curves (Fig. 1; Table 5).

Different response patterns were seen for the novel compounds tested, with IC₅₀ ranging from 7 µM to > 50 µM for KG1 and from 9 µM to > 50 µM for Molt3.

Clonogenic output of KG1 was tested in methylcellulose with 25 µM of some of the novel compounds. Colony output vs. control cultures without novel compound, was 47 % for P3, 16% for P16, 19% for P23, 8% for P27 and 0% for P28.

The novel compounds were tested for cytotoxicity on PHA-stimulated lymphocytes (PBMC), as one of the normal counterparts of the cell lines. Cells were grown for 96 hours in the presence of IL-2 and different concentrations of the novel compounds and cell number was counted on the flow cytometer. The IC₅₀ values are shown in Table 5.

**Table 5: Tested purine derivatives: structural names and IC₅₀ values on the different cell culture systems (lymphocytes, KG1, Molt3, CFU and pre-CFU) and on CDC2 activity in cell free system.**

| **Structural name** | **No.** | **Lymphocytes IC**_{**50**} **(µM)** | **KG1 IC**_{**50**} **(µM)** | **Molt3 IC**_{**50**} **(µM)** | **CFU IC**_{**50**} **(µM)** | **Pre-CFU IC**_{**50**} **(µM)** | **CDC2 IC**_{**50**} **(µM)** |
|---|---|---|---|---|---|---|---|
| | P17 | 18 ± 2.8 | 35.3 ± 5.7 | 24±6.2 | | | 1 |
| | P18 | 14.9±2.9 | 18.7± 0.3 | 23 ± 2.8 | | | 2.6 |
| 2((R)-(1-hydroxymethyl)propylamino)-6-(4-hydroxybenzylamino)-9-isopropylpurine | P19 | 6 | 11±1.3 | 15±2 | | | 1 |
| 2-(hexylamino)-6-(1-phenyl-2-hydroxyethylamina)-9-isopropylpurine | P23 | 15±2 | 8±0.6 | 16±0.8 | >50 | >25 | |
| | P29 | 9±6.6 | 16.5±1 | 9±1.2 | | | |

Comparison between normal PHA-stimulated lymphocytes and hematopoietic cell lines shows that lymphocytes are more sensitive to some of the novel compounds than cell lines, with the exception of P23, that was significantly more effective on KG1 than on normal PBMC (figure 1). To determine reversibility of the effects of the new compounds, cells were plated at 10,000 per well and exposed to 0-50 µM of the composed, either continuously for 7 days (control) or only for 6 hours. In the latter case, cells were washed after 6 hours in phosphate-buffered-saline (PBS) (Life Technologies) and reseeded into drug-free medium for 7 days. After 7 days (168 hours), cell number was assayed by flow cytometry for both culture conditions.

CD34⁺ hematopoietic progenitors (HPC) from adult bone marrow were isolated and investigated for their response to the novel compounds. CD34⁺ cells were grown in a methylcellulose system in the presence of increasing concentrations of compounds. After 14 days, colonies were microscopically scored and IC₅₀ concentrations were calculated from the dose-response patterns of total colony output (Table 5). P23 has low or no inhibitory activity on progenitors with IC₅₀ > 50 µM.

Clonogenic output of CD34⁺ HPC was also scored differentially. Culture with P23 resulted in significantly lower colony output for CFU-E, CFU-G and CFU-M.. No significant difference was seen for CFU-GM and CFU-MIX for tested compounds. Control semi-solid cultures with DMSO were not significantly different from the control cultures.

Pre-CFU were cultured starting from adult bone marrow CD34⁺CD38⁻ cells, that had been isolated using the FCM cell sorter. Novel compounds were added at different concentrations to the primary 14 day liquid culture. IC₅₀ was calculated from dose-response curves from total clonogenic output after secondary methylcellulose culture (Table 5). Effects were in a range similar to those on CFU.

### EXAMPLE 6

### Antimitotic activities of cdk inhibitors

Metaphase-arrested Xenopus egg extracts were prepared as described previously by Blow "J. Cell Biol." 1993, 122:993 and stored in liquid nitrogen. Demembranated Xenopus sperm nuclei were prepared as described. by Blow & Laskey "Cell" 1986;47:577. After thawing, extracts were supplemented with 25 mM phosphocreatine, 5 µg/ml creatine phosphokinase, 250 µg/ml cycloheximide, [α-³²P]dATP (for DNA synthesis assays). Demembranated sperm nuclei were added to a final sperm concentration of 3 ng/µl DNA extract and CDK inhibitor tested was then added at different concentrations. M-phase promoting factor inhibition by different CDK inhibitors was monitored 1.5 h after addition by assessing the amount of sperm nuclei that had been assembled into interphase nuclei, possessing a complete phase-dense nuclear envelope. DNA synthesis was assessed by releasing extract into interphase by the addition of 0.3 mM CaCl₂ and measuring the total amount of [α-³²P]dATP incorporation after 3 h by TCA coprecipitation.

At concentrations of cdk inhibitors (see Table 6) ranging from 0.1 - 2 µM, chromosomes remained highly condensed and no nuclear envelope was visible. At 4-6 µM and higher concentrations, interphase nuclei appeared with partially decondensed chromatin and an intact nuclear envelope. Replication was significantly inhibited at 1 - 5 µM CDK inhibitors tested. For the inhibition effect to become detectable, the first 15-min incubation of the interphase extract is probably sufficient.

**Table 6: Antimitotic activities of 2,6,9-trisubstituted purine derivatives**

| SUBSTITUENT | | | Inhibition of MPF activity | inhibition of DNA synthesis |
|---|---|---|---|---|
| C₂ | N6 | N9 | IC₅₀ (µM) | IC₅₀ (µM) |
| Hydroxypropylamino | 3,4-dihydroxybenzylamino | isopropyl | 3.6 | 4.2 |
| Hydroxypropylamino | [(R,S)-(1-phenyl-2-hydroxyethyl)amino] | isopropyl | 1.5 | 1.4 |
| Hydroxypropylamino | [N-(2-(3,4-dihydroxyphenyl)ethyl)-N-methyl]amino | isopropyl | 5.6 | 6.7 |
| (R)-I-(hydroxyethyl) propylamino | 3,4-dihydroxybenzylamino | isopropyl | 2.8 | 3.5 |
| (R)-1-(hydroxyethyl) propylamino | [(R,S)-(1-phenyl-2-hydroxyethyl)amino] | isopropyl | 0.9 | 1.25 |
| (R)-1-(hydroxymethyl) propylamino | [N-(2-(3,4-dihydroxyphenyl)ethyl)-N-methyl]amino | isopropyl | 1.12 | 1.3 |

### EXAMPLE 7

### In vitro cytotoxic activity of novel compounds of the invention

One of the parameters used as the basis for colorimetric assays is the metabolic activity of viable cells. For example, a microtiter assay, which uses the tetrazolium salt MTT, is now widely used to quantitate cell proliferation and cytotoxicity. For instance, this assay is used in drug screening programs and in chemosensitivity testing. Because only metabolically active cells cleave tetrazolium salts, these assays detect viable cells exclusively. In the case of MTT assay, yellow soluble tetrazolium salt is reduced to coloured water-insoluble formazan salt. After it is solubilized, the formazan formed can easily and rapidly be quantified in a conventional ELISA plate reader at 570 nm (maximum absorbance). The quantity of reduced formazan corresponds to number of vital cells in the culture.

Human T-lymphoblastic leukemia cell line CEM; promyelocytic HL-60 and monocytic U937 leukemias; breast carcinoma cell lines MCF-7, BT549, MDA-MB-231; glioblastoma U87MG cells; cervical carcinoma cells HELA; sarcoma cells U2OS and Saos2; hepatocellular carcinoma HepG2; mouse immortalized bone marrow macrophages B2.4 and B10A.4; P388D1 and L1210 leukemia; B16 and B16F10 melanomas were used for routine screening of the compounds according to the invention. The cells were maintained in Nunc/Corning 80 cm² plastic tissue culture flasks and cultured in cell culture medium (DMEM with 5 g/l glucose, 2mM glutamine, 100 U/ml penicillin, 100 µg/ml streptomycin, 10% fetal calf serum and sodium bicarbonate).

The cell suspensions that were prepared and diluted according to the particular cell type and the expected target cell density (2500-30000 cells per well based on cell growth characteristics) were added by pipette (80µl) into 96-well microtiter plates. Inoculates were allowed a pre-incubation period of 24 hours at 37°C and 5% CO₂ for stabilisation. Four-fold dilutions of the intended test concentration were added at time zero in 20 µl aliquots to the microtiter plate wells. Usually, test compound was evaluated at six 4-fold dilutions. In routine testing, the highest well concentration was 266.7 µM, but it can be the matter of change dependent on the agent. All drug concentrations were examined in duplicates. Incubations of cells with the test compounds lasted for 72 hours at 37 °C, in 5% CO₂ atmosphere and 100% humidity. At the end of incubation period, the cells were assayed by using the MTT. Ten microliters of the MTT stock solution were pipetted into each well and incubated further for 1-4 hours. After this incubation period, formazan was solubilized by addition of 100 µl/well of 10% SDS in water (pH=5.5) followed by further incubation at 37 °C overnight. The optical density (OD) was measured at 540nm with the Labsystem iEMS Reader MF (UK). The tumour cell survival (TCS) was calculated using the following equation: TCS=(OD_{drug exposed well} / mean OD_{control wells}) x 100%. The TCS₅₀ value, the drug concentration lethal to 50% of the tumour cells, was calculated from the obtained dose response curves.

The cytoxicity of novel compounds was tested on panel of cell lines of different histogenetic and species origin (Table 7). Equal activities were found in all tumour cell lines tested. Notably, identical effectiveness of purine derivatives was also found in cell lines bearing various mutations or deletions in cell cycle associated proteins, e.g. HL-60, BT549, Hela, U2OS, MDA-MB231, and Saos2. This indicates that these substances are equally effective in tumours with various alterations of tumour suppressor genes, namely p53, Rb, etc. Importantly, this observation distinguishes the purine derivatives of the invention from flavopiridol and related compounds, as their biological activity is dependent on p53 status.

**Table 7: Cytotoxicity of compounds of the invention for different cancer cells.**

| SUBSTITUENT | | | CEM | B16 |
|---|---|---|---|---|
| C2 | C6 | N9 | IC₅₀ (µM) | IC₅₀ (µM) |
| (R)-2-hydroxypropylamino | [(R,S)-(1-phenyl-2-hydroxyethyl)amino] | isopropyl | 70 | 74.6 |
| (S)-2-hydroxypropylamino | [(R,S)-(1-phenyl-2-hydroxyethyl)amino] | isopropyl | 8 | 5.6 |
| Hexylamino | [(R,S)-(1-phenyl-2-hydroxyethyl)amino] | isopropyl | 34 | 36.8 |
| (R)-1-(hydroxymethyl)propylamino | [(R,S)-(1-phenyl-2-hydroxyethyl)amino] | isopropyl | 15 | 13.5 |
| (S)-1-(hydroxymethyl)propylamino | [(R,S)-(1-phenyl-2-hydroxyethyl)amino] | isopropyl | 20 | 24 |
| (R)-2-hydroxypropylamino | 3,4-dihydroxybenzylamino | isopropyl | 35 | 41 |
| (S)-2-hydroxypropylamino | 3,4-dihydroxybenzylamino | isopropyl | 12 | 10 |
| Hexylamino | 3,4-dihydroxybenzylamino | isopropyl | 22 | 21 |
| (R)-1-(hydroxymethyl) propylamino | 3,4-dihydroxybenzylamino | isopropyl | 8 | 9 |
| (S)-1-(hydroxymethyl)propylamino | 3,4-dihydroxybenzylamino | isopropyl | 51 | 53 |
| (R)-2-hydroxypropylamino | [N-(2-(3,4-dihydroxyphenyl)ethyl)-N-methyl]amino | isopropyl | 13 | 14 |
| (S)-2-hydroxypropylamino | [N-(2-(3,4-dihydroxyphenyl)ethyl)-N-methyl]amino | isopropyl | 43 | 40 |
| Hexylamino | [N-(2-(3,4-dihydroxyphenyl)ethyl)-N-methyl]amino | isopropyl | 33 | 32 |
| (R)-1-(hydroxymethyl) propylamino | [N-(2-(3,4-dihydroxyphenyl)ethyl)-N-methyl]amino | isopropyl | 6 | 6 |
| (S)-1-(hydroxymethyl) propylamino | [N-(2'-(3.4-dihydroxyphenyl)ethyl)-N-methyl]amino | isopropyl | 36 | 34 |
| 2-(1R-isopropyl-2-hydroxyethylamino) | [N-(2-(3,4-dihydroxyphenyl)ethyl)-N-methyl]amino | isopropyl | 8 | 9 |

### EXAMPLE 8

### Novel compounds induce apoptosis in tumour cells

To analyse the mechanisms of induced cytotoxicity by novel compounds, it is important to distinguish apoptosis from the other major form of cell death, necrosis. First, at the tissue level, apoptosis produces little or no inflammation, since the neighbouring cells, especially macrophages, rather than being released into the extracellular fluid, engulf shrunken portions of the cell. In contrast, in necrosis, cellular contents are released into the extracellular fluid, and thus have an irritant affect on the nearby cells, causing inflammation. Second, at the cellular level, apoptotic cells exhibit shrinkage and blebbing of the cytoplasm, preservation of structure of cellular organelles including the mitochondria, condensation and margination of chromatin, fragmentation of nuclei, and formation of apoptotic bodies, thought not all of these are seen in all cell types. Third, at the molecular level, a number of biochemical processes take an important role in induction of apoptosis. However, majority of them is not well understood, and they result in activation of proteases and nucleases, which finally destruct key biological macromolecules - proteins and DNA. For detection of apoptotic versus necrotic mode of cell death, two independent methods were employed: assessment of morphology by fluorescence microscopy and analysis of DNA fragmentation by flow cytometry using the TUNEL technique.

### Determination of apoptosis and cell cycle distribution Microscopy:

Nuclear morphology of the cells was analysed with the fluorochromes Hoechst 33342 (λ_{Ex} max 346 nm; λ_{Em} max 460 nm) (Sigma) prepared in phosphate-buffered saline (PBS at 0.1 mg/ml, added to the culture medium at a final concentration of 2 µg/ml and ethidium bromide (EB) (λ_{Ex} max 540 nm; λ_{Sm} max 625 nm) (Sigma) prepared in PBS at 100 µg/ml and added to the culture medium at a final concentration of 2µg/ml (Lizard, 1995). Hundred cells were counted for each sample and percentage of apoptosis was determined.

### TdT-mediated dUTP nick end labeling (TUNEL):

Control and novel compound-treated cell cultures were washed with PBS and fixed in 1% buffered formaldehyde (pH 7.4) for 15 minutes on ice. After washing in PBS, cells were permeabilized in 70 % cold (-20°C) ethanol and transferred to 4 °C for at least 1 hour. After rehydratation in PBS, cells were labeled with 50 µl/well TUNEL reaction mixture (Boehringer Mannheim). Cells were incubated in this solution at 37°C for 40 minutes, washed in PBS and resuspended in 500 µl PBS containing 5 µg/ml EB and 0.1 % RNAse. After 30 minutes of incubation at 4°C, green (FITC-dUTP) and red (EB-DNA) fluorescence of individual cells was measured on a FACscan flow cytometer (Gorczyca, 1993). Negative control (fixed and permeabilized cells incubated with 50 µl label solution per well without terminal transferase, instead of TUNEL reaction mixture) and positive control (fixed and permeabilized cells incubated with DNase I (100 µg/ml) that induces DNA strand breaks) were included in each experimental set-up.

Flow cytometric detection of apoptosis combined with DNA staining can identify apoptotic cells and simultaneously evaluate their position in the cell cycle. This technique was used to provide infomation about the possible cell cycle specific initiation of apoptosis by the tested compounds. If more than 2 % of apoptotic cells were detected by the TUNEL method and the absolute number of acquired apoptotic events exceeded 2000, DNA content was simultaneously measured. Within the apoptotic population, analysis and calculation of the different cell cycle phases was carried out by an iterative curve fitting procedure (ModFit LT cell cycle analysis sofware from Verity software house, INC).

### Pro-apoptotic effect of new compounds

**Fluorescence microsopy analysis of apoptosis and necrosis:** Cell cultures treated with different doses of novel compounds ("P" number of compounds is as in Table 5 in Example 8) were examined microscopically for apoptosis. Apoptotic cells exhibit a very bright Hoechst 33342 fluorescence, while viable cells display a very faint fluorescence. Late apoptotic cells or secondarily necrotic cells display a fragmented nucleus with bright red ethidium bromide fluorescence. Primary necrotic cells show a red fluorescence and do not have fragmented nuclei.

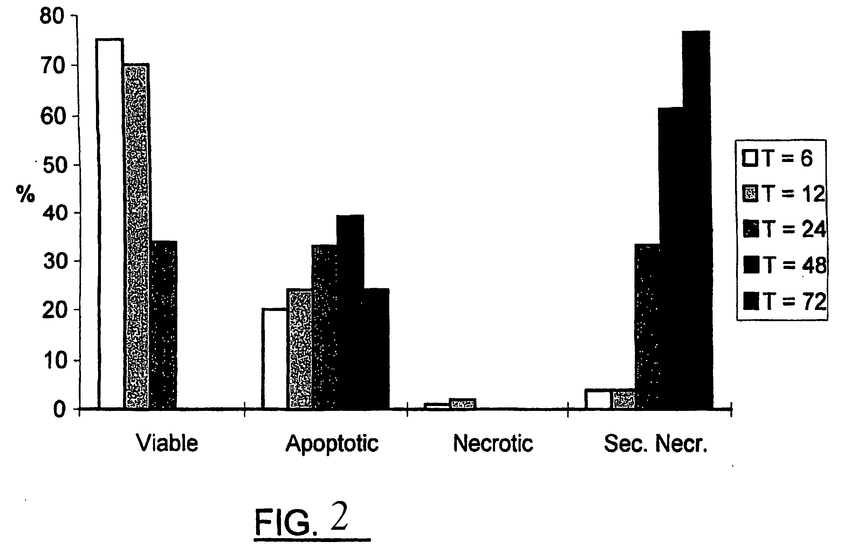

Figure 2 shows the result of microscopic examination of KG1 cells incubated with P23. Viable, apoptotic, necrotic (= primary necrosis, not following apoptosis) and secondarily necrotic cells (= late apoptotis, evolving to necrosis) were scored differentially after 6, 12, 24, 48 and 72 hours of exposure to the novel compound. For the products a different apoptosis-inducing pattern could be observed. Apoptosis induction occurs fast after incubation with P23.

Flow cytometric detection of apoptosis and cell cycle analysis: The induction of apoptotic death of KG1 cells by the novel compounds was confirmed using the TUNEL reaction technique (Fig. 5). For P23, apoptotic cell number in G₀-G₁ phase of the cell cycle is higher in comparison with the control cells all time points (untreated culture), but no significant differences were detected (Fig. 6a). Percentage of apoptotic cells in G₀-G₁ increases with time, while the number of apoptotic cells in S+G₂/M phases decreases. These differences between control and apoptotic cells were significant after 72 hours (p=0.035). No significant difference was detected between control (without incubation of product) and the non-apoptotic population in the incubated culture as measured by TUNEL. After 6 and 12 hours of incubation the apoptotic population seems mainly to evolve from S phase. Apoptotic cells in G₀-G₁ phase increase with time after 24 hours. No significant - difference was detected between control and the non-apoptotic population in the incubated culture .

### EXAMPLE 9

### Immunosuppressive activity

One of the most important parameters of specific cellular immunity is proliferative response of lymphocytes to antigens or polyclonal mitogens. The majority of normal mammalian peripheral lymphocytes comprise resting cells. Antigens or nonspecific polyclonal mitogens have the capacity to activate lymphoid cells and this is accompanied by dramatic changes of intracellular metabolism (mitochondrial activity, protein synthesis, nucleic acids synthesis, formation of blastic cells and cellular proliferation). Compounds with ability to selectively inhibit lymphocyte proliferation are potent immunosuppressants. A variety of in vitro assays was developed to measure the proliferative response of lymphocytes. The most commonly used is ³H-thymidine incorporation method.

During cell proliferation, DNA has to be replicated before the cell is divided into two daughter cells. This close association between cell doublings and DNA synthesis is very attractive for assessing cell proliferation. If labeled DNA precursors are added to the cell culture, cells that are about to divide incorporate the labeled nucleotide into their DNA. Traditionally, those assays usually involve the use of radiolabeled nucleosides, particularly tritiated thymidine ([³H]-TdR). The amount of [³H]-TdR incorporated into the cellular DNA is quantified by liquid scintillation counting.

Human heparinized peripheral blood was obtained from healthy volunteers by cubital vein punction. The blood was diluted in PBS (1:3) and mononuclear cells were separated by centrifugation in Ficoll-Hypaque density gradient (Pharmacia, 1.077 g/ml) at 2200 rpm for 30 minutes. Following centrifugation, lymphocytes were washed in PBS and resuspended in cell culture medium (RMPI 1640, 2mM glutamine, 100 U/ml penicillin, 100 µg/ml streptomycin, 10% fetal calf serum and.sodium bicarbonate).

The cells were diluted at target density of 1100000 cells/ml and were added by pipette (180 µl) into 96/well microtiter plates. Four-fold dilutions of the intended test concentration were added at time zero in 20 µl aliquots to the microtiter plate wells. Usually, test compound was evaluated at six 4-fold dilutions. In routine testing, the highest well concentration was 266.7 µM. All drug concentrations were examined in duplicates. All wells with exception of unstimulated controls were activated with 50 µl of concanavalin A (25 µg/ml). Incubations of cells with test compounds lasted for 72 hours at 37 °C, in 5% CO₂ atmosphere and 100% humidity. At the end of incubation period, the cells were assayed by using the [³H] -TdR:

Cell cultures were incubated with 0.5 µCi (20 µl of stock solution 500 µCi/ml) per well for 6 hours at 37 °C and 5% CO₂. The automated cell harvester was used to lyse cells in water and adsorb the DNA onto glass-fiber filters in the format of microtiter plate. The DNA incorporated [³H]-TdR was retained on the filter while unincorporated material passes through. The filters were dried at room temperature overnight, sealed into a sample bag with 10-12 ml of scintillant. The amount of [³H]-TdR present in each filter (in cpm) was determined by scintillation counting in a Betaplate liquid scintillation counter. The effective dose of immunosuppressant (ED) was calculated using the following equation: ED = (cpm_{drug exposed well} / mean cpm_{control vells}) x 100%. The ED₅₀ value, the drug concentration inhibiting proliferation of 50% of lymphocytes, was calculated from the obtained dose response curves.

To evaluate immunosuppressive activity of substituted adenines, their ability to inhibit polyclonal mitogen induced proliferation of normal human lymphocytes was analyzed (Table 8). Our data demonstrate that the compounds have only marginal activity on ³H-thymidine incorporation, nonetheless, they efficiently inhibit proliferation of activated lymphocytes. Effective immunosuppressive dose of new derivatives under in vitro conditions was close to 1-20 µM.

**Table 8: Immunosupressive activity of novel purine derivatives.**

| SUBSTITUENT | | | |
|---|---|---|---|
| C2 | N6 | N9 | ED₅₀ (µM) |
| (R)-2-hydroxypropylamino | [(R,S)-(1-phenyl-2-hydroxyethyl)amino] | isopropyl | 23 |
| (S)-2-hydroxypropylamino | [(R,S)-(1-phenyl-2-hydroxyethyl)amino] | isopropyl | 27 |
| Herylamino | [(R,S)-(1-phenyl-2-hydroxyethyl)amino] | isopropyl | 56 |
| 2-hydroxyethylamino | [(R,S)-(1-phenyl-2-hydroxyethyl)amino] | Isopropyl | 38 |
| (R)-1-(hydroxymethyl)propylamino | [(R,S)-(1-phenyl-2-hydroxyethyl)amino] | isopropyl | 12 |
| (S)-1-(hydroxymethyl) propylamino | [(R,S)-(1-phenyl-2-hydroxyethyl)amino] | isopropyl | 14 |
| (R)-2-hydroxypropylamino | [N-(2-(3,4-dihydroxyphenyl)ethyl)-N-methyl]amino | isopropyl | 7 |
| (S)-2-hydroxypropylamino | [N-(2-(3,4-dihydroxyphenyl)ethyl)-N-methyl]amino | isopropyl | 8 |
| Hexylamino | [N-(2-(3,4-dihydroxyphenyl)ethyl)-N-methyl]amino | isopropyl | 15 |
| (R)-1-(hydroxymethyl) propylamino | [N-(2-(3,4-dihydroxyphenyl)ethyl)-N-methyl]amino | isopropyl | 1.5 |
| (S)-1-(hydroxymethyl) propylamino | [N-(2-(3,4-dihydroxyphenyl)ethyl)-N- methyl]amino | isopropyl | 1.8 |
| 2-(IR-isopropyl-2-hydroxyethylamino) | [N-(2-(3.4-dihydroxyphenyl)ethyl-N-methyl]amino | isopropyl | 0.54 |

### EXAMPLE 10

### Dry capsules

5000 capsules, each of which contain 0.25 g of one of the purine derivatives of formula I as defined above as active ingredient, are prepared as follows:

### Composition

| | |
|---|---|
| Active ingredient | 1250 g |
| Talc | 180 g |
| Wheat starch | 120 g |
| Magnesium stearate | 80 g |
| Lactose | 20 g |

### Preparation process

The powdered substances mentioned are pressed through a sieve of mesh width 0.6 mm. Portions of 0.33 g of the mixture are transferred to gelatine capsules with the aid of a capsule-filling machine.

### EXAMPLE 11

### Soft capsules

5000 soft gelatine capsules, each of which contain 0.05 g of one of the purine derivatives of formula I as defined above as active ingredient, are prepared as follows:

### Composition

| | |
|---|---|
| Active ingredient | 250 g |
| Lauroglycol | 2 litres |

### Preparation process

The powdered active ingredient is suspended in Lauroglykol® (propylene glycol laurate, Gattefossé S.A., Saint Priest, France) and ground in a wet-pulveriser to a particle size of about 1 to 3 µm. Portions of in each case 0.419 g of the mixture are then transferred to soft gelatine capsules by means of a capsule-filling machine.

### EXAMPLE 12

### Soft Capsules

5000 soft gelatine capsules, each of which contain 0.05 g of one of the purine derivatives of formula I defined above as active ingredient, are prepared as follows:

### Composition

| | |
|---|---|
| Active ingredient | 250 g |
| PEG 400 | 1 litre |
| Tween 80 | 1 litre |

### Preparation process

The powdered active ingredient is suspended in PEG 400 (polyethylene glycol of Mr between 380 and about 420, Sigma, Fluka, Aldrich, USA) and Tween® 80 (polyoxyethylene sorbitan monolaurate, Atlas Chem. Inc., Inc., USA, supplied by Sigma, Fluka, Aldrich, USA) and ground in a wet-pulveriser to a particle size of about 1 to 3 mm. Portions of in each case 0.43 g of the mixture are then transferred to soft gelatine capsules by means of a capsule-filling machine.

## Claims

1. Purine derivatives represented by general formula I : 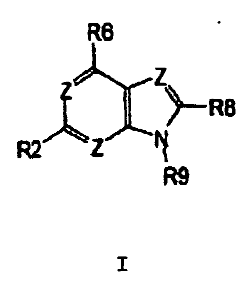 and pharmaceutically acceptable salts thereof, wherein: Z is N;
R6 is a substituted -NH-phenylalkyl, wherein alkyl is a branched or linear, saturated or unsaturated C₁-C₆ lower alkyl such as methyl, ethyl, propyl, isopropyl, vinyl, propinyl, or propenyl, wherein the phenyl ring or the alkyl is substituted by 1 to 4 substituents, said substituents for the phenyl ring being selected from hydroxyl substituents and for the alkyl independently selected from halogen, such as chloro or fluoro, hydroxyl, amino, carboxyl or amido;
R8 is H,
R2 is R2'-X wherein
X is -NH-; and
R2' is a branched or unbranched C₃-C₆ chain which may be saturated or unsaturated, such as propyl, isopropyl, tert-butyl, isobutyl, allyl, vinyl, allyl, propenyl, propargyl, propinyl, isopentenyl or isobutenyl optionally substituted with 1 to 3 substituents selected from hydroxyl, or amino; and
R9 is a branched or unbranched C₁-C₆ chain which may be saturated or unsaturated, such as for example methyl, propyl, isopropyl, tert-butyl, allyl, vinyl, ethinyl, propargyl, or hexen-2-yl.

2. Purine derivatives as claimed in claim 1, wherein the phenyl ring is substituted by 1 to 4 hydroxyl substituents.

3. Purine derivatives as claimed in claims 1-2, wherein R6 is an amine substituted with a catechol group.

4. Purine derivatives as claimed in claim 1, wherein the C₁-C₆ lower alkyl group is substituted by 1 to 4 independent substituents selected from halogen, such as chloro, fluoro, hydroxyl, amino, carboxyl or amido.

5. Purine derivatives as claimed in any of claims 1-3, wherein the purine derivatives are chosen from the group consisting of 6-(3,4-dihydroxybenzyl)aminopurine, 6-(3,4-dihydroxybenzyl)amino-9-isopropylpurine, 2-(1-hydroxymethylpropylamino)-6-(3,4-dihydroxy benzyl)-amino-9-isopropylpurine, 2-(R)-(2-hydroxymethylpyrrolidine-1-yl)-6-(3,4-dihydroxybenzyl)amino-9-ispopropylpurine, 2-(2-aminopropylamino)-6-(3,4-dihydroxybenzyl)amino-9-isopropylpurine, 2-(2-hydroxypropylamino)-6-(3,4-dihydroxybenzyl)amino-9-isopropylpurine, 2-(R)-(1-isopropyl-2-hydroxyethylamino)-6-(3,4-dihydroxybenzyl)amino-9-isopropylpurine, 6-[N-(3,4-dihydroxybenzyl)-N-methyl]amino-9-isopropylpurine, 6-[N-(3,4-dihydroxybenzyl)-N-methyl]aminopurine, 6-[N-(3,4-dihydroxybenzyl)-N-methyl]amino-8-fluoropurine, 6-[N-(3,4-dihydroxybenzyl)-N-methyl]amino-9-isopropylpurine, 2-(2-hydroxypropylamino)-6-[N-(3,4-dihydroxybenzyl)-N-methyl]amino-9-isopropylpurine, 2-(R)-(1-isopropyl-2-hydroxyethylamino-6-[N-(3,4-dihydroxybenzyl)-N-methyl]amino-9-isopropylpurine, 6-[1-(3,4-dihydroxyphenyl)ethyl]amino-9-isopropylpurine, 6-[1-(3,4-dihydroxyphenyl)ethyl]aminopurine, 6-[1-(3,4-dihydroxyphenyl)ethyl]amino-8-fluoropurine, 6-[1-(3,4-dihydroxyphenyl)ethyl]amino-9-isopropylpurine, 2-(2-hydroxy-propylamino)-6-[1-(3,4-dihydroxyphenyl)ethyl]amino-9-isopropylpurine, 2-(R)-(1-isopropyl-2-hydroxyethylamino-6-[1-(3,4-dihydroxyphenyl)ethyl]amino-9-isopropylpurine, 2-(R)-(1-isopropyl-2-hydroxyethylamino-6-[N-(2-(3,4-dihydroxyphenyl)ethyl)-N-methyl]amino-9-isopropylpurine, 6-[N-(2-(3,4-dihydroxyphenyl)ethyl)-N-methyl]amino-9-isopropy lpurine, 6-[N-(2-(3,4-dihydroxyphenyl)ethyl)-N-methyl]amino-8-bromo-9-isopropylpurine, 6-[N-(2-(3,4-dihydroxyfenyl)ethyl)-N-methyl]aminopurine, 6-(R,S)-hydroxy-1-phenylethylamino-9-isopropylpurine, 6-[N-(3,4-dihydroxybenzyl)-N-methyl]amino-9-(R)-(2-phosphonomethoxypropyl)purine, 2-Amino-6-[N-(3,4-dihydroxybenzyl)-N-methyl]amino-9-(R)-(2-phosphonomethoxypropyl)purine, 2-Amino-6-(3,4-dihydroxybenzyl)amino-9-(R)-(2-phosphonomethoxypropyl)purine, and 2-Amino-6-[N-(2-(3,4-dihydroxyphenyl)ethyl)-N-methyl]amino-9-(R }-(2-phosphonomethoxypropyl)purine, 2-(1R-isopropyl-2-hydroxyethylamino)-6-[(R)-(1-phenyl-2-hydroxyethyl)amino]-9-isopropylpurine, 2-(R)-(1-isopropyl-2-hydroxyethylamino-6)-[(R,S)-(1-phenyl-2-hydroxyethyl)amino]-isopropylpurine.

6. Method to prepare 2,6,9-trisubstituted purine derivatives of formula I in claim 1, wherein R2, R6, R9 substituents are as defined in claim 1, wherein 2-chloro-6-substituted purine derivatives having R6 substitutions as defined in claim 1 are alkylated and subsequent reacted with R2-NH.

7. Purine derivatives as claimed in any of claims 1-5 for use as an inhibitor of cyclin-dependent kinase proteins.

8. Purine derivatives as claimed in any of claims 1-5 for use as an antiviral, antimitotic, antiproliferative, immunomodulating, immune-suppressive, anti-inflammatory, antimicrobial and/or antitumor agent.

9. Purine derivatives as claimed in any of claims 1-5 for use as a modulator of α-, β-adrenergic and/or purinergic receptors.

10. Purine derivatives as claimed in any of claims 1-5 for use as an inhibitor of proliferation of hematopoietic cells and cancer cells.

11. Purine derivatives as claimed in any of claims 1-5 for use as an inducer of apoptosis in cancer cells.

12. Purine derivatives as claimed in any of claims 1-5 and 7-11 for use in treatment of the human or animal body.

13. Pharmaceutical composition comprising one or more purine derivatives as claimed in any of claims 1-5 and 7-12 and a pharmaceutically acceptable carrier or diluent.

14. Use of purine derivatives as claimed in claims 1-5 and 7-11 for the preparation of affinity absorption matrices.

15. Use of an effective amount of one or more purine derivatives as claimed in claims 1-5 and 7-12 together with a pharmaceutical acceptable carrier for the manufacturing of a medicament for inhibiting cell proliferation in mammals.

16. Use of an effective amount of one or more purine derivatives as claimed in claims 1-5 and 7-12 with a pharmaceutical acceptable carrier for the manufacturing of a medicament for the treatment of viral infections in mammals.

17. Use of an effective amount of one or more purine derivatives as claimed in claims 1-5 and 7-12 together with a pharmaceutical acceptable carrier, optionally in combination with one or more cytostatic agents for the manufactering of a medicament for the treatment of cancer in mammals.

18. Use of an effective amount of one or more purine derivatives as claimed in claims 1-5 and 7-12 together with a pharmaceutical acceptable carrier for the manufacturing of a medicament for the suppression of immune stimulation in mammals.

## Patentansprüche

1. Purinderivate dargestellt durch die allgemeine Formel I: 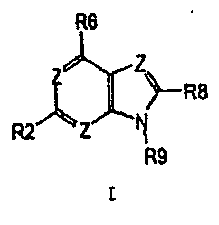 sowie pharmazeutisch akzeptable Salze davon, worin: Z N ist; R6 ein substituiertes -NH-Phenylalkyl ist, worin Alkyl ein verzweigtes oder lineares, gesättigtes oder ungesättigtes C₁-C₆-Niedrigalkyl wie Methyl, Ethyl, Propyl, Isopropyl, Vinyl, Propinyl oder Propenyl ist, wobei der Phenylring oder das Alkyl durch 1 bis 4 Substituenten substituiert ist, wobei die Substituenten für den Phenylring aus Hydroxylsubstituenten ausgewählt ist und für das Alkyl unabhängig davon aus Halogen wie Chloro oder Fluoro, Hydroxyl, Amino, Carboxyl oder Amido ausgewählt ist;
R8 H ist,
R2 R2'-X ist, worin
X -NH- ist; und
R2' eine verzweigte oder unverzweigte C₃-C₆-Kette ist, die gesättigt oder ungesättigt sein kann, wie Propyl, Isopropyl, tert-Butyl, Isobutyl, Allyl, Vinyl, Allyl, Propenyl, Propagyl, Propinyl, Isopentenyl oder Isobutenyl, wahlweise substituiert durch 1 bis 3 Substituenten, die aus Hydroxyl oder Amino ausgewählt sind;
und
R9 eine verzweigte oder unverzweigte C₁-C₆-Kette ist, die gesättigt oder ungesättigt sein kann, wie zum Beispiel Methyl, Propyl, Isopropyl, tert-Butyl, Allyl, Vinyl, Ethinyl, Propargyl oder Hexen-2-yl.

2. Purinderivate wie in Anspruch 1 beansprucht, wobei der Phenylring durch 1 bis 4 Hydroxylsubstituenten substituiert ist.

3. Purinderivate wie in den Ansprüchen 1 - 2 beansprucht, worin R6 ein mit einer Catecholgruppe substituiertes Amin ist.

4. Purinderivate wie in Anspruch 1 beansprucht, worin die C₁-C₆-Niedrigalkylgruppe durch 1 bis 4 unabhängige Substituenten substitutiert ist, ausgewählt aus Halogen wie Chloro, Fluoro, Hydroxyl, Amino, Carboxyl oder Amido.

5. Purinderivate wie in irgendeinem der Ansprüche 1 - 3 beansprucht, worin die Purinderivate ausgewählt sind aus der Gruppe, die aus 6-(3,4-Dihydroxybenzyl)aminopurin, 6-(3,4-Dihydroxybenzyl)amino-9-isopropylpurin, 2-(1-Hydroxymethylpropylamino)-6-(3,4-dihydroxybenzyl)-amino-9-isopropylpurin, 2-(R)-(2-Hydroxymethylpyrrolidin-1-yl)-6-(3,4-dihydroxybenzyl)amino-9-isopropylpurin, 2-(2-Aminopropylamino)-6-(3,4-dihydroxybenzyl(amino-9-isopropylpurin, 2-(2-Hydroxypropylamino-6-(3,4-dihydroxybenzyl)amino-9-isopropylpurin, 2-(R)-(1-Isopropyl-2-hydroxyethylamino)-6-(3,4-dihydroxybenzyl)-amino-9-isopropylpurin, 6-[N-(3,4-Dihydroxybenzyl)-N-methyl]amino-9-isopropylpurin, 6-[N-(3,4-Dihydroxybenzyl)-N-methyl]aminopurin, 6-[N-(3,4-Dihydroxybenzyl)-N-methyl]amino-8-fluoropurin, 6-[N-(3,4-Dihydroxybenzyl)-N-methyl]amino-9-isopropylpurin, 2-(2-Hydroxypropylamino)-6-[N-(3,4-dihydroxybenzyl)-N-methyl]amino-9-isopropylpurin, 2-(R)-(1-Isopropyl-2-hydroxyethylamino-6-[N-(3,4-dihydroxybenzyl)-N-methyl]amino-9-isopropylpurin, 6-[1-(3,4-dihydroxyphenyl)-ethyl]amino-9-isopropylpurin, 6-[1-(3,4-Dihydroxyphenyl)-ethyl]aminopurin, 6-[1-(3,4-Dihydroxyphenyl)ethyl]amino-8-fluoropurin, 6-[1-(3,4-Dihydroxyphenyl)ethyl]amino-9-isopropylpurin, 2-(2-Hydroxypropylamino)-6-[1-(3,4-dihydroxyphenyl)ethyl]amino-9-isopropylpurin, 2-(R)-(1-Isopropyl-2-hydroxyethylamino)-6-[1-(3,4-dihydroxyphenyl)ethyl]amino-9-isopropylpurin, 2-(R)-(1-Isopropyl-2-hydroxyethylamino-6-[N-(2-(3,4-dihydroxyphenyl)ethyl)-N-methyl]amino-9-isopropylpurin, 6-[N-(2-(3,4-Dihydroxyphenyl)ethyl)-N-methyl]amino-9-isopropylpurin, 6-[N-(2-(3,4-Dihydroxyphenyl)ethyl)-N-methyl]amino-8-bromo-9-isopropylpurin, 6-[N-(2-(3,4-Dihydroxyfenyl)ethyl)-N-methyl)aminopurin, 6-(R,S)-Hydroxy-1-phenylethylamino-9-isopropylpurin, 6-[N-(3,4-Dihydroxybenzyl)-N-methyl]amino-9-(R)-(2-phosphonomethoxypropyl)purin, 2-Amino-6-[N-(3,4-dihydroxybenzyl)-N-methyl]amino-9-(R)-(2-phosphonomethoxypropyl)purin, 2-Amino-6-(3,4-dihydroxybenzyl)amino-9-(R)-(2-phosphonomethoxypropyl)purin, und 2-Amino-6-[N-(2-(3,4-dihydroxyphenyl) ethyl)-N-methyl]amino-9-(R)-(2-phosphonomethoxypropyl)purin, 2-(1R-Isopropyl-2-hydroxyethylamino)-6-[(R)-(1-phenyl-2-hydroxyethyl)amino]-9-isopropylpurin, 2-(R)-(1-Isopropyl-2-hydroxyethylamino-6)-[(R,S)-(1-phenyl-2-hydroxyethyl)amino]-isopropylpurin besteht.

6. Verfahren zur Herstellung von 2,6,9-trisubstituierten Purinderivaten der Formel I in Anspruch 1, worin R2-, R6-, R9-Substituenten wie in Anspruch 1 definiert sind, wobei 2-Chloro-6-substituierte Purinderivate mit R6-Substitutionen wie in Anspruch 1 definiert, alkyliert werden und anschließend mit R2-NH umgesetzt werden.

7. Purinderivate wie in irgendeinem der Ansprüche 1 - 5 beansprucht zur Verwendung als Inhibitor von Cyclin-abhängigen Kinaseproteinen.

8. Purinderivate wie in irgendeinem der Ansprüche 1 - 5 beansprucht zur Verwendung als ein antivirales, antimitotisches, antiproliferatives, immunmodulierendes, immunsuppressives, antientzündliches, antimikrobielles Mittel und/oder Antitumormittel.

9. Purinderivate wie in irgendeinem der Ansprüche 1 - 5 beansprucht zur Verwendung als einem Modulator von α-, β-adrenergischen und/oder purinergenen Rezeptoren.

10. Purinderivate wie in irgendeinem der Ansprüche 1 - 5 beansprucht zur Verwendung als einen Inhibitor der Proliferation von hämatopoietischen Zellen oder Krebszellen.

11. Purinderivate wie in irgendeinem der Ansprüche 1 - 5 beansprucht zur Verwendung als einen Inducer der Apoptose in Krebszellen.

12. Purinderivate wie in irgendeinem der Ansprüche 1 - 5 und 7 - 11 beansprucht zur Verwendung bei der Behandlung des menschlichen oder tierischen Körpers.

13. Pharmazeutische Zusammensetzung, mit einem oder mehreren Purinderivat (en) wie in irgendeinem der Ansprüche 1 - 5 und 7 - 12 beansprucht und einem pharmazeutisch akzeptablen Träger oder Streckmittel.

14. Verwendung von Purinderivaten wie in den Ansprüchen 1 - 5 und 7 - 11 beansprucht zur Herstellung von Affinitätsabsorptionsmatrizes.

15. Verwendung einer wirksamen Menge von einem oder mehreren Purinderivat(en) wie in den Ansprüchen 1 - 5 und 7 - 12 beansprucht, zusammen mit einem pharmazeutisch akzeptablen Träger zur Herstellung eines Medikaments zum Hemmen der Zellproliferation bei Säugern.

16. Verwendung einer wirksamen Menge von einem oder mehreren Purinderivat(en) wie in den Ansprüchen 1 - 5 und 7 - 12 beansprucht, mit einem pharmazeutisch akzeptablen Träger zur Herstellung eines Medikaments zur Behandlung von viralen Infektionen bei Säugern.

17. Verwendung einer wirksamen Menge von einem oder mehreren Purinderivat(en) wie in den Ansprüchen 1 - 5 und 7 - 12 beansprucht, zusammen mit einem pharmazeutisch akzeptablen Träger, wahlweise in Kombination mit einem oder mehreren cytostatischen Mittel(n), zur Herstellung eines Medikaments zur Behandlung von Krebs bei Säugern.

18. Verwendung einer wirksamen Menge von einem oder mehreren Purinderivat(en) wie in den Ansprüchen 1 - 5 und 7 - 12 beansprucht, zusammen mit einem pharmazeutisch akzeptablen Träger zur Herstellung eines Medikaments zur Unterdrückung der Immunstimulation bei Säugern.

## Revendications

1. Dérivés de purine, représentés par la formule générale I : 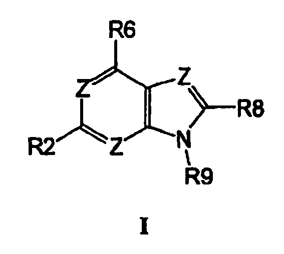 et leurs sels pharmaceutiquement acceptables,
dans laquelle Z est N
R6 est un radical -NH-phénylalkyle substitué, dans lequel le radical alkyle est un groupe alkyle inférieur en C₁ à C₆, ramifié ou linéaire, saturé ou insaturé, comme le méthyle, éthyle, propyle, isopropyle, vinyle, propinyle ou propènyle, dans lequel le cycle phényle ou le radical alkyle est substitué par 1 à 4 substituants, lesdits substituants pour le cycle phényle étant choisis parmi les substituants hydroxyle, et pour le radical alkyle, indépendamment choisis parmi les atomes d'halogènes, comme le chloro ou le fluoro, les groupes hydroxyle, amino, carboxyle ou amido ;
R8 est H,
R2 est R2'-X,
dans laquelle X est -NH- ; et
R2' est une chaîne en C₃ à C₆ linéaire ou ramifiée, qui peut être saturée ou insaturée, comme le propyle, isopropyle, tert-butyle, isobutyle, allyle, vinyle, allyle, propènyle, propargyle, propinyle, isopentènyle ou isobutènyle, le cas échéant susbtitué par 1 à 3 substituants sélectionnés parmi l'hydroxyle ou l'amino ; et
R9 est une chaîne en C₁ à C₆ linéaire ou ramifiée, qui peut être saturée ou insaturée, comme le méthyle, propyle, isopropyle, tert-butyle, allyle, vinyle, éthinyle, propargyle ou hexèn-2-yle.

2. Dérivés de purine selon la revendication 1, dans lesquels le cycle phényle est substitué par 1 à 4 substituants hydroxyle.

3. Dérivés de purine selon les revendications 1 à 2, dans lesquels R6 est une amine substituée par un groupe catéchol.

4. Dérivés de purine selon la revendication 1, dans lesquels le groupe alkyle inférieur en C₁ à C₆ est substitué par 1 à 4 substituants indépendants choisis parmi les atomes d'halogène, tels que le chloro, le fluoro, les groupes hydroxyle, amino, carboxyle ou amido.

5. Dérivés de purine selon l'une quelconque des revendications 1 à 3, dans lesquels les dérivés de purine sont choisis dans le groupe constitué par la 6-(3,4-dihydroxybenzyl)aminopurine, 6-(3,4-dihydroxybenzyl)amino-9-isopropylpurine, 2-(1-hydroxyméthylpropyl-amino)-6-(3,4-dihydroxybenzyl)amino-9-isopropylpurine, 2-(R)-(2-hydroxyméthylpyrrolidine-1-yl)-6-(3,4-dihydroxybenzyl)amino-9-isopropylpurine, 2-(2-aminopropylamino)-6-(3,4-dihydroxybenzyl)amino-9-isopropylpurine, 2-(2-hydroxypropylamino)-6-(3,4-dihydroxybenzyl)amino-9-isopropylpurine, 2-(R)-(1-isopropyl-2-hydroxyéthylamino)-6-(3,4-dihydroxybenzyl)-amino-9-isopropylpurine, 6-[N-(3,4-dihydroxybenzyl)-N-méthyl]-amino-9-isopropylpurine, 6-[N-(3,4-dihydroxybenzyl)-N-méthyl]aminopurine, 6-[N-(3,4-dihydroxybenzyl)-N-méthyl]amino-8-fluoropurine, 6-[N-(3,4-dihydroxybenzyl)-N-méthyl]amino-9-isopropylpurine, 2-(2-hydroxypropylamino)-6-[N-(3,4-dihydroxybenzyl)-N-méthyl]amino-9-isopropylpurine, 2-(R)-(1-isopropyl-2-hydroxyéthylamino)-6-[N-(3,4-dihydroxybenzyl)-N-méthyl]amino-9-isopropylpurine, 6-[1-(3,4-dihydroxyphényl)-éthyl]amino-9-isopropylpurine, 6-[1-(3,4-dihydroxyphényl)-éthyl]aminopurine, 6-[1-(3,4-dihydroxyphényl)-éthyl]amino-8-fluoropurine, 6-[1-(3,4-dihydroxyphényl)-éthyl]amino-9-isopropylpurine, 2-(2-hydroxypropylamino)-6-[1-(3,4-dihydroxyphényl)-éthyl]amino-9-isopropylpurine, 2-(R)-(1-isopropyl-2-hydroxyéthylamino)-6-[1-(3,4-dihydroxyphényl)-éthyl]amino-9-isopropylpurine, 2-(R)-(1-isopropyl-2-hydroxyéthylamino)-6-[N-(2-(3,4-dihydroxyphényl)-éthyl)-N-méthyl]-amino-9-isopropylpurine, 6-[N-(2-(3,4-dihydroxyphényl)-éthyl)-N-méthyl]amino-9-isopropylpurine, 6-[N-(2-(3,4-dihydroxyphényl)-éthyl)-N-méthyl]amino-8-bromo-9-isopropylpurine, 6-[N-(2-(3,4-dihydroxyphényl)-éthyl)-N-méthyl]amino-purine, 6-(R,S)-hydroxy-1-phényléthylamino-9-isopropylpurine, 6-[N-(3,4-dihydroxybenzyl)-N-méthyl]-amino-9-(R)-(2-phosphonométhoxypropyl)purine, 2-amino-6-[N-(3,4-dihydroxybenzyl)-N-méthyl]amino-9-(R)-(2-phosphonométhoxypropyl)-purine, 2-amino-6-(3,4-dihydroxybenzyl)-amino-9-(R)-(2-phosphonométhoxypropyl)purine, et 2-amino-6-[N-(2-(3,4-dihydroxyphényl)-éthyl)-N-méthyl]amino-9-(R)-(2-phosphonométhoxypropyl)purine, 2-(1R-isopropyl-2-hydroxyéthylamino)-6-[(R)-(1-phényl-2-hydroxyéthyl)amino]-9-isopropylpurine, 2-(R)-(1-isopropyl-2-hydroxyéthylamino-6)-[(R,S)-(1-phényl-2-hydroxyéthyl)amino]-isopropylpurine.

6. Procédé pour préparer des dérivés de purine 2,6,9-trisubstitués de formule I selon la revendication 1, dans lesquels les substituants R2, R6, R9 sont tels qu'ils sont définis dans la revendication 1, dans lesquels les dérivés de purine 2-chloro-6-substitués ayant des substitutions R6 selon la revendication 1 sont alkylés et ont subséquemment réagi avec R2-NH.

7. Dérivés de purine selon l'une quelconque des revendications 1 à 5 destinés à l'utilisation comme inhibiteur de protéines kinases cycline - dépendantes.

8. Dérivés de purine selon l'une quelconque des revendications 1 à 5 destinés à être utilisés comme agents antiviraux, antimitotiques, antiprolifératifs, immunomodulateurs, immunosuppresseurs, anti-inflammatoires, antimicrobiens et/ ou antitumoraux.

9. Dérivés de purine selon l'une quelconque des revendications 1 à 5, destinés à être utilisés comme modulateurs des récepteurs α, β-adrénergiques et/ou purinergiques.

10. Dérivés de purine selon l'une quelconque des revendications 1 à 5 destinés à être utilisés comme inhibiteurs de la prolifération des cellules hématopoïétiques et des cellules cancéreuses.

11. Dérivés de purine selon l'une quelconque des revendications 1 à 5 destinés à être utilisés comme inducteurs de l'apoptose dans les cellules cancéreuses.

12. Dérivés de purine selon l'une quelconque des revendications 1 à 5 et 7 à 11 destinés à être utilisés dans le traitement du corps animal ou humain.

13. Composition pharmaceutique comprenant un ou plusieurs dérivés de purine selon l'une quelconque des revendications 1 à 5 et 7 à 12 et un support ou diluant pharmaceutiquement acceptable.

14. Utilisation des dérivés de purine selon l'une quelconque des revendications 1 à 5 et 7 à 11 pour la préparation de matrices d'absorption d'affinité.

15. Utilisation d'une quantité efficace d'un ou de plusieurs dérivés de purine selon les revendications 1 à 5 et 7 à 12 avec un support pharmaceutiquement acceptable pour la fabrication d'un médicament destiné à l'inhibition de la prolifération cellulaire chez les mammifères.

16. Utilisation d'une quantité efficace d'un ou de plusieurs dérivés de purine selon les revendications 1 à 5 et 7 à 12 avec un support pharmaceutiquement acceptable pour la fabrication d'un médicament destiné au traitement des infections virales chez les mammifères.

17. Utilisation d'une quantité efficace d'un ou de plusieurs dérivés de purine selon les revendications 1 à 5 et 7 à 12, avec un support pharmaceutiquement acceptable, le cas échéant combinée à un ou plusieurs agents cytostatiques, pour la fabrication d'un médicament destiné au traitement du cancer chez les mammifères.

18. Utilisation d'une quantité efficace d'un ou de plusieurs dérivés de purine selon les revendications 1 à 5 et 7 à 12, avec un support pharmaceutiquement acceptable, pour la fabrication d'un médicament destiné à la suppression de la stimulation immunitaire chez les mammifères.
